# EUROPEAN PATENT SPECIFICATION

(11) **EP 4 106 620 B1**
(45) Date of publication and mention of the grant of the patent: **28.10.2026**
(21) Application number: 21757818.6
(22) Date of filing: 17.02.2021
(51) Int. Cl.: A61B 5/02, A61B 5/00, A61B 5/0215, A61B 5/026, A61B 5/145, A61F 2/07, A61F 2/82, A61F 2/958

(54) **VASCULAR SENSING SYSTEM**
VASKULÄRES SENSORSYSTEM
SYSTÈME DE DÉTECTION VASCULAIRE

(30) Priority: 17.02.2020 US 202062977633 P; 22.01.2021 US 202163140760 P
(43) Date of publication of application: 28.12.2022
(73) Proprietor: Canary Medical Switzerland AG, 6340 Baar (CH)
(72) Inventor: ADLER, Mark A., Carlsbad, California 92010 (US)
(74) Representative: Forresters IP LLP
(86) International application number: PCT/US2021/018387
(87) International publication number: WO 2021/167983

(56) References cited:
- WO-A1-2007/057739
- WO-A2-2007/138578
- US-A1- 2003 004 403
- US-A1- 2005 165 317
- US-A1- 2008 119 911
- US-A1- 2008 176 271
- US-A1- 2008 275 350
- US-A1- 2012 197 349
- US-A1- 2013 053 711
- US-A1- 2014 180 323
- US-A1- 2017 328 931
- US-A1- 2019 076 033
- US-A1- 2019 104 936

## Description

### BACKGROUND

### Field

The present disclosure relates generally to medical devices with at least one sensor and methods of using such devices and the data generated therefrom.

### Description of the Related Art

At the present time, it is common for a health care professional to deliver a medical device to a patient, with the expectation that the medical device will function properly and provide a therapeutic benefit to the patient. Whether the medical device is actually functioning properly, or is about to miss-function, can be very difficult to determine. Likewise, whether the medical device is providing a consistent therapeutic benefit to the patient, can be very difficult to determine. There thus remains a need in the art for an accurate and sensitive method for determining how well an implanted medical device, such as a stent, is performing.

Documents US 2013/053711 A1, WO 2007/138578 A2, WO 2007/057739 A1 and US 2008/119911 A1 disclose implantable sensor assemblies.

At the present time, it is also common for a health care professional to diagnose a problem in a patient but have no convenient way to monitor that problem over time. There thus remains a need in the art for an accurate and sensitive method for determining how an undesirable condition in a patient is progressing or regressing.

The present disclosure addresses one or both of these needs.

All of the subject matter discussed in the Background section is not necessarily prior art and should not be assumed to be prior art merely as a result of its discussion in the Background section. Along these lines, any recognition of problems in the prior art discussed in the Background section or associated with such subject matter should not be treated as prior art unless expressly stated to be prior art. Instead, the discussion of any subject matter in the Background section should be treated as part of the inventor's approach to the particular problem, which in and of itself may also be inventive.

### SUMMARY

In brief, in one aspect, the present disclosure provides an independent system for use in conjunction with vascular lesion treatment in the coronary, peripheral and carotid human arterial or venous vessels. The system may include one or more features as described herein.

In one aspect, the system can comprise an assembly that can be delivered by a guidewire, providing for a percutaneous introduction of the system into the body. The system can make use of the standard lumen delivery style percutaneous delivery system design, either over the wire (OTW) or rapid exchange.

In one aspect, the system can comprise a loading and release system, which in one aspect can be configured as a balloon expandable release system, while in another aspect is configured as an unsheathing system (similar to self-expanding stent release system), while in yet another aspect is configured as an integrated ferrule locking mechanism/release system.

In one aspect, the system can comprise different integrated communicating capabilities, which may be any of physical, electronic or tactile communicating capabilities, so that information from the system may be received by an interested party, such as a doctor. Such information may inform the interested party about, for example, the status of an implanted component of the assembly, such status being, for example, informative about whether there is proper location and/or placement of the assembly prior to any additional placement or treatment to correct the disease (e.g., lesion or blockage) in the cardiovascular system.

In one aspect, the system can comprise integrated sensing capability, and in one aspect can include a pressure sensor. In another aspect, the system can comprise a vibration sensor.

In one aspect, the system can provide an independent and agnostic cardiovascular sensing system that can be deployed prior to the standard treatment methods for blocked cardiovascular arteries, and placed in the zone of the lesion for treatment, placing sensors that can monitor blood and vessel specificity to manage the acute and long term biologic reaction to the treatment zone communicating information for analytical management and decision processing to an external or internal receiving station.

The invention is directed toward an implantable sensor assembly as defined in claim 1 that can be used to monitor a treatment site of a patient. In addition or alternatively, the circuitry can include circuitry for powering and/or recharging the implantable sensor assembly, and/or circuitry for processing data collected from the one or more sensors.

The implantable sensor assembly of the preceding paragraphs or as described further herein can also include one or more of the following features. The circuitry extends either from the first sensor to the second sensor or from the first anchor to the second anchor. For example, the circuitry can be a wire that connects the first sensor and/or first anchor to the second sensor and/or second anchor. In some configurations, the circuitry can extend from the first sensor to the second sensor. The circuitry can include an antenna. The implantable sensor assembly can be configured to detect a wakeup signal from the one or more external devices and to be activated in response to detecting the wakeup signal. For example, the implantable sensor assembly can include a wakeup receiver configured to detect the wakeup signal and to activate the sensor assembly in response to detecting the wakeup signal. In some configurations, the circuitry can include the wakeup receiver or be configured to detect the wakeup signal and to activate the sensor assembly in response to detecting the wakeup signal.

At least one of the first sensor or the second sensor can be a blood flow sensor, a blood pressure sensor, a metabolic sensor, a glucose sensor, a pressure sensor, an oxygen sensor, and/or a protein enzyme sensor. Each of the first and second anchors can be configured to expand from a first diameter in a delivery configuration to a second diameter in a deployed configuration. For example, the first and second anchors can be configured to be loaded into a delivery system and/or a delivery device when in the delivery configuration. Each of the first and second anchors can be a tacking stent that can have a length less than or equal to about 9 mm. For example, the first and second anchors may have the same length or different lengths. At least one of the first anchor or second anchor can include a plurality of struts and a plurality of cells. The plurality of cells can be positioned between the plurality of struts. At least one cell of the plurality of cells can be sized and configured to receive a sensor. For example, a cell of the first anchor can receive the first sensor and a cell of the second anchor can receive the second sensor. The sensors can be configured to be coupled to a crown of the plurality of struts. The one or more sensors can be configured to be coupled to an edge of the first anchor or the second anchor. For example, the first sensor can be coupled to the edge of the first anchor and the second sensor can be coupled to the edge of the second anchor. The edges of the first and second anchors coupled to the first and second sensors can face toward or away from the treatment site.

The circuitry can be configured to wirelessly transmit raw data collected from the one or more sensors. At least one of the one or more sensors can form a part of a sensor system including processing circuitry configured to at least partially process the sensor data collected from the one or more sensors. The circuitry can be configured to wirelessly transmit the at least partially processed sensor data. The circuitry can be configured to wirelessly receive instructions from the one or more external devices. The implantable sensor assembly can be configured to receive power from the one or more external devices. For example, the implantable sensor assembly can receive power via the circuitry. The implantable sensor can further comprise a power source configured to provide power to the sensor assembly. The power source can be rechargeable. The power source can be configured to receive power from the one or more external devices. For example, the circuitry can receive power from the one or more external devices and provide the power to the power source. The power source can include a battery or a capacitor. The power source can be hermetically sealed. The sensor assembly can be configured to be powered by a power source outside the patient.

The one or more characteristics can include pressure, flow, sound, vibration, or appearance of the environment surrounding the implantable sensor assembly (e.g., the environment of the treatment site). The sensor can be hermetically sealed. The implantable sensor assembly can further comprise a unique identification code comprising information about the implantable sensor assembly. The unique identification code can be configured to be scanned by a barcode scanner. The unique identification code can be integrated with a RFID. The implantable sensor assembly can further comprise a memory device for storing the sensor data related to the one or more characteristics. The communications circuitry can be configured to wirelessly communicate with the one or more external devices via a Bluetooth^{™} protocol, WiFi, ZigBee, medical implant communication service ("MICS"), the medical device radio communications service ("MedRadio"), or cellular telephony.

A kit including the implantable sensor assembly of any of the preceding paragraphs and/or any of the implantable sensor assemblies described herein is disclosed. The kit can include a delivery system configured to deliver the sensor assembly to the body passageway of the patient (e.g., the treatment site). The delivery system can be a balloon catheter. The delivery system can include a sheath configured to cover the first and second anchors when delivering the implantable sensor assembly to the body passageway of the patient.

Certain aspects of this disclosure are directed toward a sensor assembly that can be implanted into a body passageway of a patient and used to monitor the body passageway of the patient. The sensor assembly can include at least one anchor, at least one sensor, and circuitry. The at least one anchor can be configured to be positioned on at least one side of a treatment site of the body passageway. For example, the at least one anchor can include first and second anchors. The first anchor can be configured to be positioned on a first side of a treatment site of the body passageway of the patient. The second anchor can be configured to be positioned on a second side of the treatment site. The at least one sensor can configured to collect sensor data related to one or more characteristics of the environment surrounding the sensor assembly when implanted in the body passageway. For example, the environment surrounding the sensor assembly can be at or near the treatment site. The at least one anchor can be configured to carry the at least one sensor. For example, the at least one sensor can include a first sensor and a second sensor. The first anchor can carry a first sensor and a second anchor can carry a second sensor. The circuitry can be configured to wirelessly communicate with an external device outside of the body of the patient. Additionally or alternatively, the circuitry can include circuitry for powering and/or recharging the implantable sensor assembly, and/or circuitry for processing data collected from the one or more sensors.

The sensor assembly of the preceding paragraphs or as described further herein can also include one or more of the following features. The sensor assembly can further comprise a power supply configured to provide power to the sensor assembly. The power supply can be rechargeable. The power supply can be coupled to the circuitry. The power supply can be configured to receive power from the one or more external device via the circuitry. The power supply can be a battery or a capacitor. The power supply can be hermetically sealed. The sensor assembly can be configured to receive power from the one or more external devices. The sensor assembly can be configured to be powered by a power source outside the patient. The circuitry can be configured to receive instructions from outside the patient. For example, the circuitry can receive system updates for the sensor assembly.

Each of the first anchor and the second anchor can be configured to expand from a first diameter in a delivery configuration to a second diameter in a deployed configuration. Each of the first anchor and the second anchor can be a tacking stent that can be no longer than 9 mm. The one or more characteristics can include comprises pressure, flow, sound, vibration, or appearance of the environment surrounding the sensor assembly. For example, the environment can be the body passageway of the patient, such as at or near the treatment site of the patient.

The circuitry can extend from the first anchor to the second anchor. The circuitry can include an antenna extending from the first anchor to the second anchor. The sensor assembly can be configured to detect a wakeup signal from the one or more external devices and to be activated in response to detecting the wakeup signal. For example, the sensor assembly can include a wakeup receiver configured to detect the wakeup signal and to activate the sensor assembly in response to detecting the wakeup signal. In some configurations, the circuitry can include the wakeup receiver or be configured to detect the wakeup signal and to activate the sensor assembly in response to detecting the wakeup signal.

The at least one sensor can be hermetically sealed. The sensor assembly can further comprise a unique identification code comprising information about the sensor assembly. The unique identification code can be configured to be scanned by a barcode scanner. The unique identification code can be integrated with a RFID. The at least one sensor can include a blood flow sensor, a blood pressure sensor, a metabolic sensor, a glucose sensor, a pressure sensor, an oxygen sensor, and/or protein enzyme sensor. The at least one sensor can include a first sensor and a second sensor. For example, the first anchor can be configured to carry the first sensor and the second anchor can be configured to carry the second sensor. At least one of the first anchor or second anchor can include a plurality of struts and a plurality of cells between the plurality of struts. At least one cell of the plurality of cells can be sized and configured to receive the at least one sensor. For example, a cell of the first anchor can receive the first sensor and a cell of the second anchor can receive the second sensor. The at least one sensor can be configured to be coupled to a crown of the plurality of struts. The at least one sensor can be configured to be coupled to an edge of the first anchor or the second anchor. For example, the first sensor can be coupled to the edge of the first anchor and the second sensor can be coupled to the edge of the second anchor. The edges of the first and second anchors coupled to the first and second sensors can face toward or away from the treatment site.

The sensor assembly can further comprise a memory device for storing sensor data related to the one or more characteristics. The at least one sensor can form a part of a sensor system can including a processor configured to at least partially process the sensor data collected from the environment surrounding the sensor assembly. The circuitry can be configured to transmit raw data collected by the at least one sensor. The circuitry can be configured to wirelessly communicate with the one or more external devices via a Bluetooth^{™} protocol, WiFi, ZigBee, medical implant communication service ("MICS"), the medical device radio communications service ("MedRadio"), or cellular telephony.

A kit including the sensor assembly of any of the preceding paragraphs and/or any of the sensor assemblies described herein is disclosed. The kit can include a delivery system configured to deliver the sensor assembly to the body passageway of the patient (e.g., the treatment site). The delivery system can be a balloon catheter. The delivery system can include a sheath configured to maintain the first and second anchors in a delivery configuration. Each of the first and second anchors can include a first diameter when in the delivery configuration. The first and second anchor can be configured to expand from the first diameter in the delivery configuration to the second diameter in a deployed configuration when the sensor assembly is deployed from the sheath.

Certain aspects of this disclosure are directed toward a sensor assembly. The sensor assembly can be used to monitor a treatment site and/or a body passageway of the patient. The sensor assembly can include at least one anchor, at least one sensor, and a power capacity system. The at least one sensor can include a first sensor and a second sensor. In some configurations, the at least one sensor can include more than two sensors. For example, the at least one sensor can include four sensors, six sensors, eight or more sensors. The at least one anchor can include a first anchor and a second anchor. The first anchor can carry the first sensor or more than one sensor and the second anchor can carry the second sensor or more than one sensor. The at least one sensor can be configured to collect sensor data related to one or more characteristics of the body passageway and/or the treatment site of the patient. The power capacity system can include circuitry configured to wirelessly communicate with one or more external devices.

The sensor assembly of the preceding paragraphs or as described further herein can also include one or more of the following features. The power capacity system can extend from the first sensor to the second sensor. The power capacity system can extend from the first anchor to the second anchor. The power capacity system can include an antenna. The sensor assembly can be configured to detect a wakeup signal from the one or more external devices and to be activated in response to detecting the wakeup signal. For example, the sensor assembly can include a wakeup receiver configured to detect the wakeup signal and to activate the sensor assembly in response to detecting the wakeup signal. In some configurations, the power capacity system can include the wakeup receiver or be configured to detect the wakeup signal and to activate the sensor assembly in response to detecting the wakeup signal.

The sensor assembly can be configured to receive power from the one or more external devices. For example, the sensor assembly can receive power from the one or more external devices via the power capacity system. The power capacity system can comprise a power supply configured to provide power to the sensor assembly. The power supply can be rechargeable. The power capacity system can be configured to receive power from the one or more external devices and deliver power to the power supply. The power supply can include a battery or a capacitor. The power supply can be hermetically sealed. At least one of the first sensor and the second sensor can be a blood flow sensor, a blood pressure sensor, a metabolic sensor, a glucose sensor, a pressure sensor, an oxygen sensor, and/or a protein enzyme sensor. Each of the first and second anchors can be configured to expand from a first diameter in a delivery configuration to a second diameter in a deployed configuration. Each of the first and second anchors can be a tacking stent that can have a length less than or equal to about 9 mm.

The power capacity system can be configured to wirelessly transmit raw data collected from the at least one sensor. The at least one sensor can be part of at least one sensor system including processing circuitry configured to at least partially process the sensor data collected from the at least one sensor. In some configurations, the power capacity system can include the processing circuitry or be configured to at least partially process the sensor data collected from the at least one sensor. The power capacity system can be configured to wirelessly transmit the at least partially processed sensor data. The power capacity system can be configured to wirelessly receive instructions from the one or more external devices. The one or more characteristics can include pressure, flow, sound, vibration, or appearance of the environment surrounding the sensor assembly.

The at least one sensor system can be hermetically sealed. In some configurations, the sensor assembly can be hermetically sealed or at least some components of the sensor assembly can be hermetically sealed. The sensor assembly can further comprise a unique identification code comprising information about the sensor assembly. The unique identification code can be configured to be scanned by a barcode scanner. The unique identification code can be integrated with a RFID. The sensor assembly can further comprise a memory device for storing the sensor data related to the one or more characteristics. The power capacity system can be configured to wirelessly communicate with the one or more external devices via a Bluetooth^{™} protocol, WiFi, ZigBee, medical implant communication service ("MICS"), the medical device radio communications service ("MedRadio"), or cellular telephony.

A kit including the sensor assembly of any of the preceding paragraphs and/or any of the sensor assemblies described herein is disclosed. The kit can include a delivery system configured to deliver the sensor assembly to the body passageway of the patient. The delivery system can be a balloon catheter. The delivery system can include a sheath configured to cover the first and second anchors when delivering the sensor assembly to the body passageway of the patient.

Certain aspects of this disclosure are directed toward a method of implanting a sensor assembly into a lumen of a patient. The method can include: advancing a delivery system carrying a sensor assembly to the lumen of the patient; deploying the first anchor on a first side of a treatment site; deploying the second anchor on a second side of the treatment site; and/or removing the delivery system from the patient. The second side of the treatment site can be opposite the first side. The sensor assembly can include at least one anchor, one or more sensors, and circuitry. The at least one anchor can be configured to expand from a delivery configuration to a deployed configuration. The at least one anchor can include a first anchor and a second anchor. The first and second anchors can be connected. The one or more sensors can be configured to collect sensor data related to one or more characteristics of the lumen. The one or more sensors can be carried by the first anchor and the second anchor. The circuitry can be configured to wirelessly communicate with one or more external devices.

The method of the preceding paragraphs or as described further herein can also include one or more of the following features. The method can further include expanding a balloon of the delivery system to expand the first anchor and/or the second anchor. The method can further include deploying a treatment device at the treatment site. The treatment device can be a stent. The treatment device can be deployed in the lumen before advancing the delivery system to the lumen. The method can further include creating a false lumen within a wall of the lumen adjacent to the treatment site. The method can further include positioning the circuitry through the false lumen. The method can further include positioning the first anchor on a first side of the false lumen and positioning the second anchor on a second side of the false lumen. The method can further include deploying the circuitry through the lumen adjacent the treatment site. The circuitry can be deployed before deploying the second anchor.

The method can further include wirelessly transmitting the sensor data related to the one or more characteristics to the one or more external devices. The method can further include wirelessly receiving instructions from the one or more external devices. The method can further include receiving power from the one or more external devices. The one or more characteristics can include pressure, flow, sound, vibration, or appearance of the environment surrounding the sensor assembly. The one or more sensors can include a first sensor and a second sensor. The first sensor can be carried by the first anchor and the second sensor can be carried by the second anchor.

Certain aspects of this disclosure are directed toward a method of implanting a sensor assembly into a lumen of a patient. The method can include: creating a false lumen in a wall of the lumen of the patient; advancing a delivery system carrying a sensor assembly through the false lumen; deploying the first anchor in the lumen on a first side of the false lumen; deploying the second anchor in the lumen on a second side of the false lumen; and/or removing the delivery system from the patient. The second side of the false lumen can be opposite the first side of the false lumen. The sensor assembly can include at least one anchor, one or more sensors, and circuitry. The at least one anchor can be configured to expand from a delivery configuration to a deployed configuration. The at least one anchor can include first and second anchors. The first and second anchors can be connected. The one or more sensors can be configured to collect sensor data related to one or more characteristics of the lumen. The one or more sensors can be carried by the first anchor and the second anchor. The circuitry can be configured to wirelessly communicate with one or more external devices.

The method of the preceding paragraphs or as described further herein can also include one or more of the following features. The method can further include expanding a balloon of the delivery system to expand the first anchor and/or the second anchor. The method can further include deploying a treatment device in the lumen of the patient. The treatment device can be a stent. The treatment device can be deployed in the lumen before creating the false lumen. The method can further include positioning the circuitry through the false lumen. The circuitry can be positioned in the false lumen before deploying the second anchor. The method can further include wirelessly transmitting sensor data related to the one or more characteristics to the one or more external devices. The method can further include wirelessly receiving instructions from the one or more external devices. The method can further include receiving power from the one or more external devices. The one or more characteristics can include pressure, flow, sound, vibration, or appearance of the environment surrounding the sensor assembly. The one or more sensors can include a first sensor and a second sensor. The first anchor can be configured to carry the first sensor and the second anchor can be configured to carry the second sensor.

Certain aspects of this disclosure are directed toward an assembly for implantation into a body passageway of a patient. The assembly can include one or more anchors, one or more sensors, a transmitter, and a power supply. Each of the anchors can have a diameter. Each anchor can be configured to expand from a delivery diameter to a larger deployed diameter. Each anchor can include a deployed state. Each anchor can abut an inner wall of the body passageway and hold the assembly in a fixed location when in the deployed state. The one or more sensors can be configured to detect and measure a characteristic of an environment surrounding the implanted assembly. The transmitter can extend between the one or more anchors. For example, the one or more anchors can include two anchors. The transmitter can extend between the two anchors. The transmitter can be configured to: (i) transmit data or information from the implanted assembly to a location outside of the body of the patient; (ii) receive instructions from a location outside of the body of the patient; and/or (iii) receive power. The power supply can provide power to the assembly.

The assembly of the preceding paragraphs or as described further herein can also include one or more of the following features. The one or more sensors can be hermetically sealed. The power supply can be hermetically sealed. Each of the anchors or one of the anchors can be a tacking stent. The one or more sensors can be configured to detect and measure at least one of pressure, flow, sound, vibration, and appearance of the environment surrounding the implanted assembly.

A kit including the assembly of any of the preceding paragraphs and/or any of the assemblies described herein is disclosed. The kit can include a unique identification code. The kit can include a balloon catheter. The kit can include a guidewire.

Certain aspects of this disclosure are directed toward a method of implanting the assembly of the preceding paragraphs and/or any of the assemblies described herein into a lumen of a patient is disclosed. The method can include: advancing a guidewire to a desired location in a lumen of the body passageway of the patient; advancing a balloon catheter along the guidewire to the desired location; expanding the balloon on the balloon catheter to expand the two anchors so that the two anchors contact the inner wall of the lumen and thereby affix the anchors and the assembly in the desired location; and/or deflating the balloon and removing the balloon catheter. The balloon catheter can be joined to the assembly. The balloon catheter can include a balloon. The desired location can be a lesion of a blood vessel. The method can further include deploying a therapeutic stent to the site of the lesion to treat the lesion. The two anchors of the assembly can be located distal to and proximal to the treatment stent. The assembly can be deployed within the blood vessel before the therapeutic stent is deployed at the site of the lesion. The therapeutic stent can be deployed at the site of the lesion before the assembly is deployed within the blood vessel. The desired location can be a chronic total occlusion (CTO) of a blood vessel. The method can further include creating a false lumen within a wall of the blood vessel adjacent to the CTO. The two anchors of the assembly can be located distal to and proximal to the CTO while the transmitter runs through the false lumen.

Certain aspects of this disclosure are directed toward a method of implanting the assembly of the preceding paragraphs and/or any of the assemblies described herein into a lumen of a patient is disclosed.

Certain aspects of this disclosure are directed toward a method of determining one or more characteristics of an environment in the vicinity of a selected location in a body passageway. The method can include providing an assembly of the preceding paragraphs and/or any of the assemblies described herein; implanting the assembly at the selected location; sensing one or more characteristics of the environment in the vicinity of the implanted assembly; and/or transmitting data or information related to the one or more characteristics of the environment to a location outside of the body of the patient. The information can be obtained by processing the data related to the one or more characteristics of the environment.

The above-mentioned and additional features of the present disclosure and the manner of obtaining them will become apparent, and the assembly will be best understood by reference to the following more detailed description.

The details of one or more embodiments are set forth in the description below. The features illustrated or described in connection with one exemplary embodiment may be combined with the features of other embodiments. Other features, objects and advantages will be apparent from the description, the drawings, and the claims.

### BRIEF DESCRIPTION OF THE DRAWINGS

Exemplary features of the present disclosure, its nature and various advantages will be apparent from the accompanying drawings and the following detailed description of various embodiments. Non-limiting and non-exhaustive embodiments are described with reference to the accompanying drawings, wherein like labels or reference numbers refer to like parts throughout the various views unless otherwise specified. The sizes and relative positions of elements in the drawings are not necessarily drawn to scale. For example, the shapes of various elements are selected, enlarged, and positioned to improve drawing legibility. The particular shapes of the elements as drawn have been selected for ease of recognition in the drawings. One or more embodiments are described hereinafter with reference to the accompanying drawings in which:
FIG. 1 shows an example assembly of the present disclosure implanted in the true lumen of a blood vessel that is being treated for stenosis, including the treatment device (e.g., a treating stent) and an example delivery system including a guidewire and a balloon catheter.
FIG. 2 shows an example assembly of the present disclosure delivered via a false lumen of a blood vessel in order to evaluate a chronic total occlusion (CTO).
FIG. 3 provides a context diagram of an example assembly environment in a patient's home.

### DETAILED DESCRIPTION

The various embodiments may be understood more readily by reference to the following detailed description of preferred embodiments of the invention and the Drawings and Examples included herein. This detailed description is organized into various sections. Any headings used within this document are only being utilized to expedite its review by the reader, and should not be construed as limiting the embodiments or claims in any manner. Thus, the headings and Abstract of the Disclosure provided herein are for convenience only and do not interpret the scope or meaning of the embodiments.

The present detailed description contains the following sections:
I. Overview of Aspects of the Present Disclosure
II. Components of the Assembly
III. Coupling of the Components to form the Assembly
IV. Optional Components
V. Deploying the Assembly
VI. Operating the Assembly
VII. Communication with the Assembly
VIII. Additional Specific Exemplary Embodiments

In reading this detailed description, and unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. The term "comprises" means "includes." The abbreviation, "e.g." is derived from the Latin exempli gratia, and is used herein to indicate a non-limiting example. Thus, the abbreviation "e.g." is synonymous with the term "for example."

### I. OVERVIEW OF ASPECTS OF THE PRESENT DISCLOSURE

In one aspect, the present disclosure provides an assembly which may be fixedly positioned within a body passageway of a patient in order to gather and provide relevant information. In one aspect, the assembly does not provide any therapeutic benefit to the patient other than gathering and providing relevant information that optionally may be used to design or modify a treatment regimen that can afford a therapeutic benefit to the patient. The assembly of the present disclosure may have various functional features including, e.g., not limited to, an anchor to secure the assembly in place within a body passageway, a sensing capacity or sensor to detect and/or measure the local environment where the assembly has been deployed, a power supply to provide the power needed by the assembly to operate as envisioned, and/or a transmitter or circuitry to send out information obtained by the sensor (sometimes referred to herein as communications circuitry) and/or to receive power that can be used to charge the power supply. The components of the assembly that can provide these functional features can be coupled to one another either directly or indirectly. Some or all of the components that provide these functional features may be placed within a hermetically sealed container, and the assembly may have more than one hermetically sealed container.

In one aspect, the present disclosure provides a coupling of various components to provide an assembly of the present disclosure. For example, in one aspect, the assembly can comprise two anchors and a transmitter or circuitry that can be in the form of a wire (e.g., an antenna), which can run from one anchor to the other anchor, i.e., between the two anchors. In one aspect, the assembly can comprise two anchors, each of which can be associated with one of the two sensing capabilities or sensors, i.e., the assembly has two pairs of one anchor coupled to one sensing capability or sensor. In one aspect, the assembly can comprise two power sources, each of which can be associated with a different sensing capability or sensor. In one aspect, the assembly can comprise an anchor that can be coupled to both of a sensing capability or sensor and a power source. In one aspect, the assembly can comprise an anchor that can be coupled to both of a sensing capability or sensor and a power source, where circuitry can run between and can be coupled to each of the two anchors.

In one aspect, the present disclosure provides a system or kit that can include the assembly of the present disclosure and one or more auxiliary items. Example auxiliary items can include one or more of (i) a bar code scanner to identify the assembly, where this identification may optionally be associated with other details pertinent to the patient that receives the implant; or (ii) a balloon catheter to assist in delivering and deploying the assembly.

In one aspect, the present disclosure provides a method of deploying the assembly of the present disclosure. For example, a guidewire may be inserted into a body passageway, where the guidewire can be extended to a desired location within the passageway. A balloon catheter, onto which the assembly can be joined, can be deployed along the guidewire to the desired location. Upon reaching the desired location, the balloon can be inflated, thus expanding the anchors and fixing them into place on either side of a location of interest, e.g., a lesion. The balloon can be deflated, and the catheter and guidewire can be removed. As another example, a false lumen may be created in the vessel wall adjacent to the CTO, and the assembly, which can be joined to a balloon catheter, can be inserted through the false lumen to the extent that the leading anchor travels past the CTO and exits the false lumen to be deployed in the occluded lumen on a first side of the CTO. The trailing anchor may not enter the false lumen, but instead can be deployed in the occluded lumen on a second side of the CTO with the second side being opposite the first side. In this way, an anchor can be located on either side of the CTO, with the antenna running through the false lumen.

These and other aspects of the present disclosure are described in further detail herein.

### II. COMPONENTS OF THE ASSEMBLY

### A. Anchor

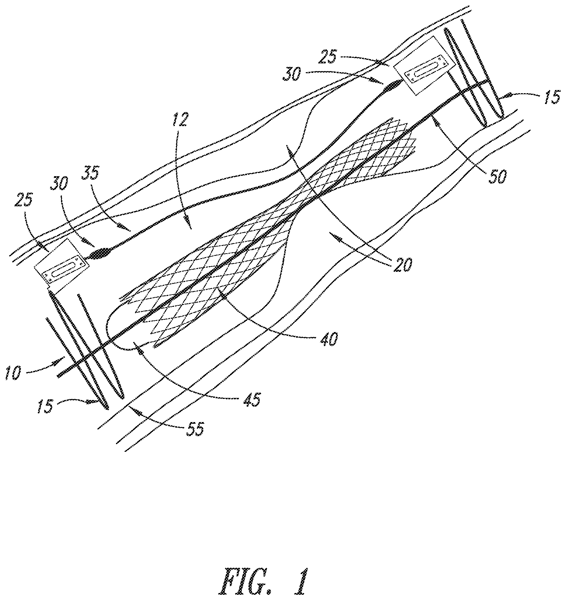

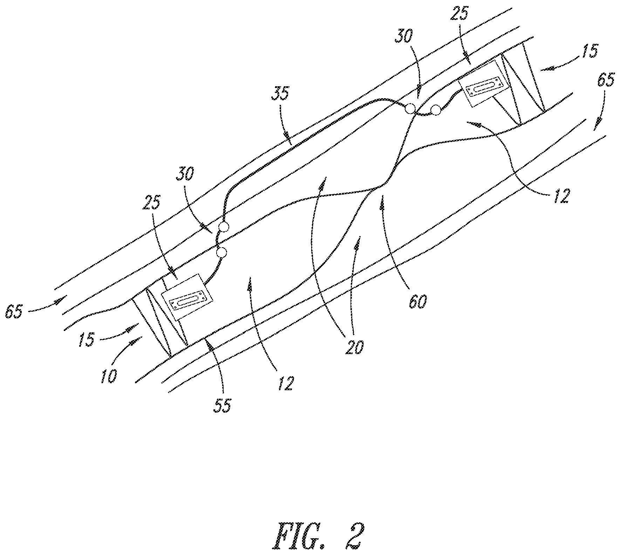

In one aspect, the assembly 10 of the present disclosure can include two features that provide for anchoring of the assembly 10 within a body passageway, where these features are referred to herein as anchors 15, which are shown in Figures 1 and 2 described below. The two anchors 15 may be the same or different, but each can have the ability to stay fixed in place after the assembly 10 has been delivered and deployed at the desired location of a body passageway. In an assembly 10 of the present disclosure, an antenna 35 can run between the two anchors 15, and reporting networks (comprising one or more of a sensor and/or a sensor system 25, a power source 30, a circuit and other features necessary or desirable to the operation of the assembly 10) are directly or indirectly coupled to one or both of the anchors 15.

In one aspect, the anchor 15 can be a tacking stent, which may also be referred to as a staking stent. A tacking stent 15 can be essentially a very short stent that can have a deployed length on the order of less than 10 mm, e.g., 6-8 mm. For example, the deployed length can be between about 1 mm and about 10 mm, about 2 mm and about 8 mm, about 4 mm and about 6 mm, or less than 9 mm. A tacking stent 15 may be made from the same materials as a stent that is designed to treat a lesion 12, and may be deployed in the same manner as a stent that is designed to treat a lesion.

The staking stent 15 may be made of an implantable material that can be used to fuse or bond the sensors or sensor system 25 and antenna 35, placing in a confined or unconfined volume of fluid or space which can be tacked into place by a deploying mechanism or a releasing mechanism with the staking stent having super-elastic properties.

Example materials from which the staking stent 15 may be made include stainless steel 316 or 17-7, cobalt-chrome, MP35N, nickel-titanium (nitinol), titanium and tantalum.

As discussed above, the implantable sensor assembly 10 can include multiple anchoring features 15 configured to anchor the implantable assembly 10 at a desired location within a body passageway of a patient. The multiple anchoring features 15 can include first and second anchors 15. In some configurations, the anchors 15 can be connected. For example, the circuitry 35 can extend between the two anchors 15 and/or the circuitry 35 can connect the two anchors 15. In some configurations, at least one of the anchors 15 can be directly or indirectly coupled to the one or more sensors 25 and/or the communications circuitry 35. For example, the sensor 25 may be encapsulated by the anchor 15. The anchor 15 may have an open cell configuration such that a cell of the anchor 15 can be sized to receive the sensor 25. When the sensor 25 is positioned within the cell of the anchor 15, one or more struts and crowns of the anchor 15 can surround the sensor 25. For example, the strut(s) and crown(s) of the anchor 15 may provide radial and axial protection of the sensor system 25 during deployment. At least one side of the sensor 25 may be covered by the strut(s) and/or the crown(s). For example, the sensor 25 may be coupled to one or more struts and/or crowns. In some configurations, the sensor 25 can be coupled to the end of the anchor 15.

The anchor 15 can have super-elastic properties such that the anchor stent 15 can have a delivery configuration and a deployed configuration. The anchor 15 can have a first diameter in the delivery configuration and a second diameter in the deployed configuration. The first diameter may be smaller than the second diameter. For example, the first diameter of the anchor 15 can be sufficient to fit within a delivery system (e.g., a balloon catheter 45). The second diameter of the anchor 15 may be sufficient to anchor the implantable sensor assembly 10 to the desired location.

### B. (B) Sensing Capacity/ Sensor(s)

### 1. Sensor(s)

In one aspect, the assembly 10 of the present disclosure can include a sensor and/or sensor system 25 (sometimes referred to herein as a "sensing component") which can afford the assembly 10 with a sensing capacity. For example, the sensor system 25 can refer to the combination of one or more sensors. In some examples, one or more sensors can be combined with processing circuitry for at least partially processing the collected sensor data (e.g., filtering, conditioning, converting, and/or calculating) to form the sensor system. As mentioned previously, a sensor 25 can refer to one or more sensors, e.g., one sensor 25, or a plurality of sensors 25 such as two, three, four, five, six, seven, eight, or more than eight sensors 25. Moreover, a sensor system 25 can refer to one or more sensor systems 25, e.g., one sensor system 25, or a plurality of sensor system 25 such as two, three, four, five, six, seven, eight, or more than eight sensor systems 25. When the assembly 10 is implanted in a patient, the sensor 25 can detect a status or situation present within the patient but outside of the assembly 10, where that status can be typically characteristic of an environment within the vicinity of the implanted assembly 10, and the sensor can make measurements that characterize that status or situation.

In one aspect, the assembly 10 of the present disclosure can include a sensor 25 selected from a pressure sensor, a sound sensor, a vibration sensor, an optical sensor, and a fluid flow pressure, where a pressure sensor can detect and measure pressure, a sound sensor can detect and measure sound, etc. The assembly 10 may have a mixture of sensors 25, i.e., the sensors 25 of the assembly 10 may be a mixture of different kinds of sensors, e.g., both of a pressure sensor and a sound sensor may be components of the assembly 10, where a pressure sensor can refer to one or more pressure sensors and a sound sensor can refer to one or more sound sensors.

In one aspect, the sensor 25 can be able to detect and measure pressure. In one aspect, the sensor 25 can be able to detect and measure pressure and can include a sensor 25 that can detect and measure sound (e.g., a microphone). In one aspect, the sensor 25 can be able to detect and measure both pressure and vibration. The measure of vibration may be achieved, e.g., by an accelerometer. A measurement of vibration may be used to correlate with vessel wall fibrillation. In one aspect, the assembly 10 can include sensors 25 that can detect and measure each of pressure, sound and vibration. In one aspect, the sensor 25 can include optical sensing, for example, in the blue and green light wavelengths (465 nm to 570 nm wavelength). Optionally, the sensor 25 can include one or more sensing capabilities selected from pressure sensing, sound sensing, vibration sensing and optical sensing. These sensing capabilities can enable the detecting and measuring of multiple physiological outputs from the human cardiovascular system, which can be used to achieve various desirable goals as described herein. In one aspect, the assembly 10 can utilize a pressure, fluid flow and/or microphone all in conjunction or single use based on the desired monitoring of physiological need in the cardiovascular vessel.

### C. Power Supply

In one aspect, the assembly 10 of the present disclosure can include a power supply 30. The power supply 30 can provide power to, e.g., the sensor and/or the sensor system 25, and also to the transmitter or circuitry 35 so that information can be transmitted from the assembly 10. In some configurations, the power supply 30 can be directly or indirectly coupled to the anchors 15. The sensor system(s) 25 can each include a power supply 30 and/or the circuitry 35 can include a power supply 30. In some configurations, the assembly 10 can be configured to be powered by a power source outside the assembly 10 and, for example, outside the patient when the assembly 10 is implanted in the patient.

In one aspect, the power supply 30 can be selected from a supercapacitor (supercap) and an ultracapacitor. Supercapacitors and ultracapacitors are commercially available from several sources, where particularly small ones that can be suitable for use in the assemblies 10 of the present disclosure may be obtained from, e.g., Seiko Instruments USA (Torrance CA, USA).

In one aspect, the power supply 30 can be battery, which may optionally be a rechargeable battery. The battery may be, for example, a small hermitic battery that can be recharged by inductance.

Optionally, the power supply 30 can be a combination of a super cap and a battery, that may work in conjunction to provide a hybrid super capacitor battery such that recharge and run times are balanced to be efficient for the required monitoring power and communication drain.

### D. Transmitter/Communications Circuitry

In one aspect, the assembly 10 of the present disclosure can include a transmitter 35. In one aspect, the transmitter 35 can send out information obtained by the sensor 25 of the assembly 10. In another aspect, the transmitter 35 can function to receive power that can then be delivered to, stored, and distributed from the power supply 30. Optionally, the transmitter 35 can perform both of these functions. The transmitter 35 may alternatively be referred to as an antenna or communications circuitry 35.

In one aspect, the transmitter 35 can be in the form of a wire or tube. The transmitter 35 may be formed from metal or metal alloy, for example, a metal selected from gold and platinum, or an alloy of platinum and iridium, e.g., an alloy of 90% platinum and 10% iridium (weight basis). When in a wire or tubular shape, the cross-sectional area of the transmitter 35 may range from about 10⁻⁶ to 10⁻² inches squared (in²), e.g., from about 0.000001 inches squared to about 0.008 inches squared. In one aspect, the cross-sectional area can be about 0.000001 in² to about 0.00001 in². In one aspect, the cross-sectional area can be about 0.00001 in² to about 0.0001 in². In one aspect, the cross-sectional area can be about 0.0001 in² to about 0.001 in². In one aspect, the cross-sectional area can be about 0.0001 in² to about 0.001 in².

In one aspect, the transmitter 35 can be a single strand of wire or tube, i.e., a monofilament. In one aspect, the transmitter 35 ca be a multifilament formed from two or more monofilaments combined, e.g., in a winded or braided or coiled configuration. The multifilament form can provide increased density compared to the corresponding monofilament, which may enhance the receipt and transmission of signals to support the transfer of information in a single or multi directional direction.

As previously mentioned, the transmitter or communications circuitry 35 can extend from the first anchor 15 to the second anchor 15 and/or from a first sensor 25 to a second sensor 25. For example, the communications circuitry 35 can directly or indirectly connect the first and second anchors 15 and/or the first and second sensors25. The communications circuitry 35 can be positioned in the true lumen 12 (Figure 1) or a false lumen (Figure 2) of a blood vessel. In some configurations, the communications circuitry 35 in conjunction with the anchors 15 can be configured to send information obtained by the sensor or sensor system 25 to external devices. For example, the communications circuitry 35 can comprise an antenna and the anchors 15 can be a part of the antenna.

### E. Electronics Assembly

The assembly 10 of the present disclosure can include a sensing component 25 (e.g., a sensor and/or sensor system) that may be part of an electronics assembly component. The electronic assembly component may include a printed circuit board assembly (PCBA) including a substrate which may be rigid, flexible, or a combination of rigid and flexible substrates. The printed circuit board (PCB) can mechanically support and electrically connect electronic components using conductive tracks, pads and other features etched from copper sheets laminated onto a non-conductive substrate.

An example electronics assembly may have a microcontroller unit (MCU). An MCU is a small computer on a single metal-oxide-semiconductor (MOS) integrated circuit chip. It can be similar to, but typically less sophisticated than, a system on a chip (SoC); however a SoC may include a microcontroller as one of its components. The MCU may be built onto a single printed circuit board, where this board provides all of the circuitry necessary for a useful control task: microprocessor, I/O circuits, clock generator, RAM, stored program memory and any support ICs necessary.

An example electronics assembly may have a power source, which can direct power though an optional fuse to a main power supply.

An example electronics assembly may have a microcontroller unit (MCU) in communication with a real time clock (RTC) module, and also in communication with a medical implant communication service (MICS) radio (the radio can be in further communication with an antenna), where the RTC module may send information to the MICS radio. A wake up signal may be sent to the MCU by either of the RTC module or the MICS radio.

The MCU may also be in communication with a memory, e.g., a non-volatiles (FLASH) memory. The memory can store the data and/or information obtained by the sensors and/or sensor systems 25.

The MCU may also be in communication with an inertial measurement unit (IMU). Optionally, the circuit board can provide sampling and communication abilities that allow the IMU to be sampled at precise intervals synchronized to Global Positioning System (GPS) pulses. The data can be minimally processed on-board and returned to a separate processor for inclusion in an overall system. The circuit board can allow the normal overhead associated with IMU data collection to be performed outside of the system processor, freeing up time to run intensive algorithms in parallel.

Optionally, the IMU can be in communication with a buck converter. A buck converter is a DC-to-DC power converter which steps down voltage from its input to its output. It is a class of switched-mode power supply that can contain at least two semiconductors and at least one energy storage element (e.g., a capacitor, inductor, or the two in combination).

Optionally, the IMU can be in communication, via a serial wire, to one or more programming pads.

Taken together, the sensor and the associated electronics assembly may be referred to as an implantable reporting processor (IPR) or a sensor system. The IPR can be a component of the assembly 10 of the present disclosure, where the assembly 10 can comprise the IPR, the antenna 35 and the anchors 15. The power supply 30 may or may not be a component of the IPR.

### III. COUPLING OF THE COMPONENTS TO FORM THE ASSEMBLY

### A. Mechanical Coupling of Components

In one aspect, the present disclosure provides a coupling of various components to provide an assembly 10 of the present disclosure. For example, in one aspect, the assembly 10 can comprise two anchors 15 and a transmitter 35 in the form of a wire, e.g., an antenna, which can run from one anchor 15 to the other anchor 15, i.e., between the two anchors 15. In one aspect, the assembly 10 can comprise two anchors 15, each of which can be associated with one of the two sensing capabilities 25, i.e., the assembly 10 can have two pairs of one anchor 15 coupled to one sensing capability 25. In one aspect, the assembly 10 can comprise two power sources 30, each of which can be associated with a different sensing capability 25. In one aspect, the assembly 10 can comprise an anchor 15 that can be coupled to both of a sensing capability 25 and a power source 30. In one aspect, the assembly10 can comprise an anchor 15 that can be coupled to both of a sensing capability 25 and a power source 30, where an antenna 35 can run between and can be coupled to each of the two anchors 15.

In one aspect, components may be fused, bonded or integrated to the anchor 15, e.g., a tacking stent. The anchor 15 may be coupled to the other components of the assembly 10 by the following example methods. For example, each of the anchors 15 can be fused by - utilizing an acoustic, a laser or a secondary energy source. The mechanical attachment can be formed through similar material fusing, primary and secondary material fusing, by plating, coating or flowing a material to cross over the joint. The material can be gold, platinum, or shim material similar to the tacking stent material, or a combination thereof. As another example, each of the anchors 15 can be bonded utilizing an implantable polymer - the implantable polymer may be, for example, a heat shrinkable TFE, PTFE, poly-ethylene or parylene, which may be processed to connect through melting, heat shrinking or retaining an adhesive.

### B. Formation of the Electronics Assembly

The electronic assembly may be formed in a multi-step process. That process may include one or more of the following steps: inner layer; drilling; baking (production), a set of steps including one or more of deburring, desmear, PTH and panel plating, followed by a second panel plating; dry film/image transfer; etching; etching check; solder mask; C/M printing; gold plating; surface finishing; punching; and cleaning. The finished assembly can be checked for quality in a quality control process including one or more of electrical testing and visual inspection.

### C. Hermetic Seal

In one aspect, the assembly 10 or selected components thereof can be contained within a hermetic seal. In this way, the assembly 10 or selected components thereof may not come into direct contact with the body of the patient within whom the assembly is implanted. Negating direct contact with the body of the patient is advantageous in order that little to no undesirable reaction can occur between the sealed assembly 10 or portions thereof, and the patient's body. For example, fluid (e.g., water or water with dissolved ions) from the patient's body may not contact and possibly degrade vulnerable components of the assembly 10. Likewise, an undesirable bodily response in the patient, which can be due to contact between the patient's body and a feature of the assembly 10, can be reduced or avoided if that feature is behind a hermetic seal.

In one aspect, the sensor of the assembly 10 can be exposed to the patient's body when the assembly 10 is implanted in the patient. In this situation, the hermetic seal can preclude direct contact between the patient's body and components of the assembly 10 while at the same time providing a window through which the sensor 25 can sense and detect what is happening outside the hermetic seal. The nature of that window, and accordingly the features of the hermetic seal which provide that window, depend on the input required by the sensor 25.

Optionally, the hermetic seal may be created by wrapping the assembly 10 or selected components thereof with a non-permeable material. In one aspect, the non-permeable material can be a metal, optionally in the form of a film to provide a metallic film (thin-film). In one aspect, the hermetic seal is can be formed from a woven cloth or polymer and metallic coated to ensure non-permeability and assure non-transmission of fluids such as water or blood.

Fig. 1 shows an example assembly 10 of the present disclosure that can be implanted in the true lumen 12 of a blood vessel that is being treated for stenosis, including a treating device 40 (e.g., a stent) and a delivery system 45 that can include a guidewire and a balloon catheter. In Fig. 1, the assembly 10 includes two anchors 15 each can be in the form of a tacking stent, which can be positioned on either side of a treatment site 20 (e.g., a lesion) that can cause a narrowing of the lumen 12. Coupled to each anchor 15 can be a sensor or sensor system 25 and a power supply (also referred to as a power cell) 30. In addition, the assembly 10 can include an antenna 35 that can run between the two anchors 15. Also shown in Fig. 1 is a treating stent 40 that can be delivered by a balloon catheter 45 to the site of the lesion 20. The balloon catheter 45, in turn, can be guided to the lesion 20 by use of a guidewire 50. A similar or the same guidewire 50 may be used to guide the assembly 10 to the site of the lesion 20 for deployment. In Fig. 1, the anchor 15 is shown abutting the vessel wall 55 and thus the anchor 15 is shown in an expanded or deployed form.

Fig. 2 shows an example assembly 10 of the present disclosure can be delivered via a false lumen of a blood vessel in order evaluate a chronic total occlusion (CTO) 60. In Fig. 2, the assembly 10 can include two anchors 15 that can each be in the form of a tacking stent, which can be positioned on either side of a chronic total occlusion 60, which can be formed when the lesions 20 become so large that they totally block the lumen 12 of the blood vessel. Coupled to each anchor 15 can be a sensor or sensor system 25, which can include a pressure sensor or any sensor as described herein, and a power supply 30 which in Fig. 2 is shown as a supercap however could be any power supply 30 as described herein. In addition, the assembly 10 can include an antenna 35 that can run between the two anchors 15 through a false lumen formed in the wall 65 of the blood vessel. In Fig. 2, the assembly 10 is shown in a deployed state, and Fig. 2 does not show the delivery vehicles for the assembly 10. In Fig. 2, the anchor 15 is shown abutting the vessel wall 55 and thus the anchor 15 is shown in an expanded or deployed form.

### IV. OPTIONAL COMPONENTS

The present disclosure provides kits and systems that include the assembly 10 of the present disclosure, in combination with one or more items. The one or more items may be associated with the assembly 10 in order to assist in, for example, the deployment of the assembly 10, to facilitate the operation of the implanted assembly 10, and/or to complement the function of the implanted assembly 10, e.g., the item may be a therapeutic stent which can treat a lesion 20, where the therapeutic stent may or may not have its own sensors.

For example, to facilitate the deployment of the assembly 10 as described herein, the present disclosure provides a kit that can include the assembly 10 and a delivery device or system (e.g., a balloon catheter 45). In another aspect, the present disclosure provides a kit that can include the assembly 10 and a guidewire, optionally with a balloon catheter 45 also being present in the kit. In another aspect, the present disclosure provides a kit that can include the assembly 10 as described herein and a unique identification code that can be specific for the assembly 10 within the kit. Optionally, this identification code can be read by a barcode scanner. The unique identification code may be integrated with a RFID. The identification code may be part of a kit that also includes one or both of a balloon catheter and a guidewire.

In some configurations, the kit can include a base station, a receiving scanner, a receiver transmitter, and/or a receiving card. In operation, the sensor data obtained from the sensor or sensor system 25 can be transmitted from the assembly 10, optionally after storage in a memory present as part of the assembly 10. An integrated Bluetooth^{™}, galvanic coupling or radio may be a component of the antenna communication system 35 or integrated to the sensor or sensor system 25 itself such that a link up (i.e., a communication data package) can be transmitted through the patient (e.g., a chest cavity of the patient) to the receiving scanner, the receiver transmitter, the receiving card (similar to a EKG port contact), and/or the base station. Data may be transferred from the receiver transmitter to the upload cloud database and communicated to the necessary receiving equipment.

### V. DEPLOYING THE ASSEMBLY

As shown in Figures 1 and 2, the assembly 10 can be deployed in the cardiovascular system of a patient. For example, if the anchors 15 of the assembly 10 are tacking stents, the assembly 10 may be positioned on a balloon, and delivered and deployed to a site in the cardiovascular system and used to deliver and deploy a stent 40 to a location in a body passageway. For example, the assembly 10 may be delivered via assistance from a guidewire, providing for a percutaneous introduction of the assembly 10 into the body. The assembly 10 of the present disclosure may be delivered percutaneously by an over the wire (OTW) or rapid exchange (RX) delivery system (using balloon expandable or self-expanding sheath release system), either prior to or after the diagnosed lesion 20 being treated cardiovascular stenting.

In one aspect, the system can comprise a loading and release system, which in one aspect can be configured as a balloon expandable release system, while in another aspect can be configured as an unsheathing system (similar to self-expanding stent release system), while in yet another aspect can be configured as an integrated ferrule locking mechanism/release system.

In the case where the assembly 10 will be deployed in conjunction with the treatment of a lesion, the assembly 10 including the anchors 15 thereof, may not interfere with that treatment. For example, if the assembly 10 is deployed in conjunction with a stent 40 to treat a vascular lesion, then the anchors 15 of the assembly 10 can be located on either side of the stented lesion, i.e., proximal and distal to the lesion 20, and may not be within the lesion 20 or the stented region of the lesion 20. A minimum distance between an edge of the lesion 20 and an edge of the anchor 15 may be described in terms of the diameter of the body passageway (e.g., a blood vessel). In one aspect, there is at least one diameter of distance between the edge of the lesion 20 and the edge of the anchor 15. In other aspects, there is at least two diameters, or three diameters, or four diameters, or five diameters of distance between an edge of the lesion 20 and an edge of an anchor 15, where optionally the reference to at least, may be change to exactly or approximately. In this way, the anchors 15 can be sufficiently spaced apart from the lesion 20 such that the anchors 15 may not treat the lesion 20, aggravates the lesion 20, or interferes with the functioning of the treatment device 40 that treats the lesion 20, while being sufficiently close to the lesion 20 and associated treatment device 40 that the sensor or sensor system(s) 25 coupled to the anchors 15 can provide useful information about the local environment of the treatment device 40 and/or the lesion 12.

In one aspect, the present disclosure provides the following procedural methodology comprising one or more of the following steps. 1. Patent preparation and lesion identification; 2. Preparing of the assembly 10 for implantation; 3. Determination if CTO or non-CTO deployment is to be used; 4a. If CTO deployment is necessary, use, e.g., the CrossBow^{™} system and/or Stingray^{™} LP, each available from Boston Scientific(Marlborough, MA, USA) and designed for CTO situations, to thereby create a false lumen in the artery wall next to the lesion area; followed by delivery of the assembly 10 of the present disclosure over the guidewire installed with the CrossBow/Stingray system which can be ultimately deployed distal and proximal in the false lumen, then deliver the device 40 for lesion treatment, cross through with the assembly 10 of the present disclosure, and then treat the lesion 4b. If non-CTO deployment is available, deliver a device for lesion treatment, typically using a guidewire, cross through with the assembly 10 of the present disclosure; 5. Confirm the assembly of the present disclosure has connectivity to a reader system, and establish a baseline reading.

The method of deploying the assembly 10 can include the following steps. For example, the assembly 10 may be loaded onto a delivery device or system. In one aspect, the delivery system can comprise a loading and release system that can be a balloon expandable release system (e.g., a balloon catheter 45), an unsheathing system (e.g., a self-expanding stent release system), or an integrated ferrule locking mechanism/release system. The illustrated configuration in Figure 1 shows a balloon catheter 45 with a guidewire 50. The clinician can determine if the lesion 12 has resulted in a CTO 60 (Figure 2). If a CTO 60 has not been formed (Figure 1), the assembly 10 can positioned on a balloon of the balloon catheter 45 and delivered to a desired site (e.g., a site of a lesion 20) via the true lumen 12 of the blood vessel. The first anchor 15 and/or the first sensor 25 can be deployed on a first side of the lesion 20 and the second anchor 15 and/or the second sensor 25 can be deployed on a second side of the lesion 20. The circuitry 35 can be deployed along the true lumen 12 after the first anchor 15 is deployed. During the deployment of the first and second anchors 15, the balloon of the balloon catheter 45 can be inflated to expand the first and second anchors 15 until the first and second anchors 15 abut against vessel wall, thereby anchoring the assembly 10 to the desired site. The balloon catheter 45 may be used to deliver and deploy a treatment device 40 (e.g., a therapeutic stent) to the desired site in the cardiovascular system of the patient. The assembly 10 may be delivered prior to or after the therapeutic device 40. The assembly 10 may be delivered percutaneously by an OTW or RX delivery system, which can utilize a balloon expandable or self-expanding sheath release system. After the assembly 10 has been deployed to the desired site, connectivity between a reader system and the assembly 10 can be confirmed and a baseline reading can be established.

If a CTO 60 has been formed (Figure 2), a false lumen can be created in the vessel wall 65 adjacent the CTO 60 prior to delivering the assembly 10. For example, the delivery system can include the CrossBow^{™} system or Stingray^{™} LP system. The delivery system can be used to create the false lumen in the vessel wall 65 next to the lesion 12 and/or CTO 60 area and deliver the assembly 10 over the guidewire of the delivery system. For example, the delivery system can be used to deliver the first or distal anchor 15 and/or the first sensor 25 through the false lumen and to the true lumen 12 such that the first anchor 15 and/or the sensor 25 can be deployed to a first or distal side of the false lumen and/or the CTO 60. The circuitry 35 can be deployed through the false lumen. The second anchor 15 and/or the second sensor 25 can be deployed to a second or proximal side of the false lumen and/or the CTO 60 such that the circuitry 35 of the assembly 10 can extend through the false lumen with the first and second anchors 15 and/or the first and second sensor 25 positioned in the true lumen 12. The delivery system can be delivered through the second or proximal anchor 15 to deploy the treatment device 40 between the first or distal anchor 15 and the second or proximal anchor 15.

### VI. OPERATING THE ASSEMBLY

### A. Data Acquisition and Generation

As mentioned previously, the sensor and/or the sensor system 25 of an implanted assembly 10 can detect an environmental situation, typically a local environmental situation, i.e., a situation characteristic of the environment in the immediate vicinity of the sensor, and/or make measurements characterizing that environmental situation. The detection can generate data, and the measurement can likewise generate data.

The sensor data may include a series of measurements taken over a time period, e.g., a plurality of measurements taken over a second, a few seconds, or a longer period of time. The measurements may take place periodically, e.g., every few seconds a measurement can be taken, or every few seconds a plurality of measurements can be taken. In some configurations, the measurements may take place in response to instructions received from an external device.

As one example, the sensor may detect and/or measure the pressure being exerted upon the assembly 10 by the patient. For instance, the sensor may detect and/or measure a blood pressure that exists within a blood vessel in the vicinity of the sensor.

### B. Data Evaluation

The assembly 10 of the present disclosure can acquire data that is descriptive of the local environment within which the assembly 10 has been implanted in the patient. For example, the assembly 10 can be configured to collect data related to one or more characteristics of a body passageway within the patient. The sensor data may be processed to provide information, where the data (e.g., raw data) and/or information (e.g., processed data) may be evaluated by an interested party, such as a health care provider or a stent manufacturer, to obtain an understanding of what is happening, at a particular point in time, within the vicinity of the assembly 10.

The data and/or information, which may be collectively referred to for convenience as information, may be characteristic of the baseline state of the patient. For example, the information may be indicative of the well-being (e.g., status) of the patient, and/or of the assembly 10, and/or of the patient/assembly 10 interaction, during periods of repetitive events (e.g., when sleeping or when walking). The baseline can be determined by making measurements during multiple instances of the repetitive event, e.g., each evening for multiple evenings, data can be obtained characteristic of the patient's status while sleeping. This data can collectively provide a baseline descriptor of the patient, and/or of the assembly 10, and/or of the patient/assembly 10 interaction, while sleeping. The sleeping baseline can be useful in order to provide a comparator to data obtained at a later date to see whether the status within the patient at the later date has deviated from the sleeping baseline situation as determined during an earlier period of time. An interested party may evaluate whether the deviation suggests that a modality of care should be implemented or revised in order to better serve the interests of the patient.

In one aspect, a baseline can be obtained and the modality of care for a patient can be purposely changed. For example, after the baseline has been obtained, a patient may change their activity level, ingest certain medications, and/or make other changes to their lifestyle or treatment regimen. After this change, data can be obtained pertaining to the patient, the assembly 10, and/or to the patient/assembly 10 interaction. The data may be used to assess the impact of the change. Based on this assessment, the change may be curtailed, maintained, revised, etc., as determined by the judgment of the health care provider. In view of the change, a new baseline may be obtained.

In one aspect, a baseline can be obtained and the sensor 25 can subsequently and periodically generate data that can be compared to the baseline. If the post-baseline data deviates from the baseline, the interested party may use that observation to understand what is happening at the site of assembly implantation, and can make adjustments to the treatment regimen, next generation assembly designs.

After implantation of a treatment device 40, bio-fouling or a reaction to the treatment device 40 may occur. These problems may give rise to changes in sensor output of the sensor 25 relative to a baseline. Based on these changes in sensor output of the sensor 25, the interested party may be able to determine the problem that has given rise to the change in sensor output of the sensor 25 and take corrective action.

The sensor 25 may be used to detect acute variables of hydrodynamic wave patterns, which could be disrupted by embolic materials or density pattern changes in fluid. A density change could relate to embolic transmission, a plasma density change can be based on dietary intake (non-saturated fats or organic tissue and cholesterol changes).

### VII. COMMUNICATION WITH THE ASSEMBLY

The assembly 10 may be part of an environment which can communicate with the assembly 10. An example environment can be an operating room wherein the assembly 10 can be implanted into a patient by a health care profession. Another example environment can be the patient's home, in the case where the assembly 10 has already been implanted in the patient. Yet another example environment can be a doctor's office, where the patient having the implanted assembly 10 is in the office for, e.g., an evaluation. The following provides a detailed description of an example environment being a patient's home. However, the described features and connectivity are analogously present in other assembly environments within which the patient with the implanted assembly 10 is present, e.g., the operating room and the doctor's office, as also described herein albeit in lesser detail.

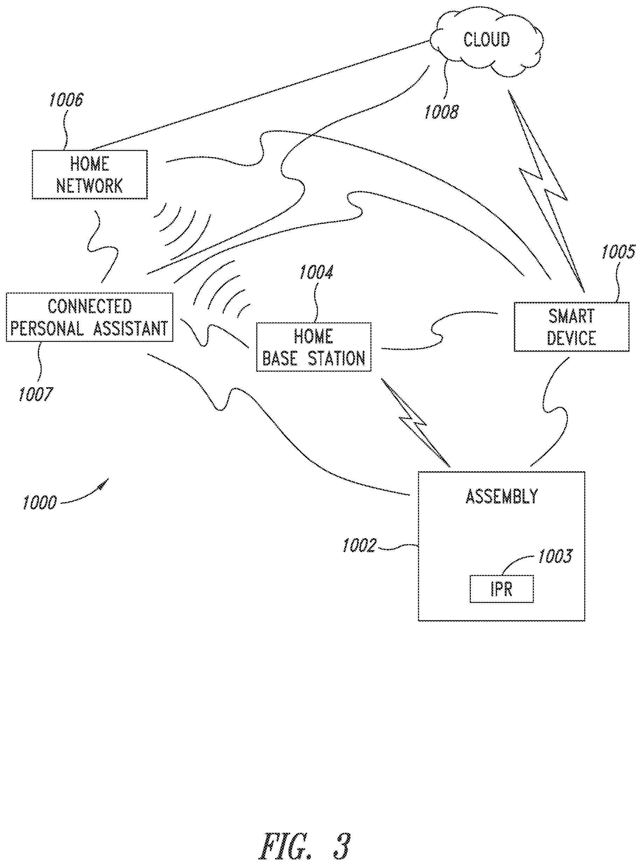

Fig. 3 illustrates a context diagram of an assembly environment 1000 including features present in the patient's home. In the environment 1000, an assembly 1002 comprising an implantable reporting processor (IPR) 1003 has been implanted into a patient (not shown). The assembly 1002 can be the same or similar to the assembly 10 described above in relation to Figures 1 and 2. The IPR 1003 can be the same or similar to the sensor system 25 described above in relation to Figures 1 and 2. The IPR 1003 can be arranged and configured to collect data. For example, the data can include including medical and health data related to the patient which the device is associated with, and/or operational data of the implantable device 1002 itself. The assembly 1002 can be configured to communicate with one or more home base stations 1004 or one or more smart devices 1005 during different stages of monitoring the patient.

The assembly 1002 can include one or more sensors that collect information and data, including medical and health data related to a patient which the device 1002 is associated with, and operational data of the implantable device 1002 itself. The assembly 1002 can collect data at various different times and at various different rates during a monitoring process of the patient, and may optionally store that the collected data in a memory until it can be transmitted to one or more external devices outside the body of the patient. In some embodiments, the assembly 1002 may operate in a plurality of different phases over the course of monitoring the patient. For example, more data can be collected soon after the assembly 1002 is implanted into the patient, but less data can be collected as the patient heals and thereafter.

The amount and type of data collected by the assembly 1002 may be different from patient to patient and/or the amount and type of data collected may change for a single patient. For example, a medical practitioner studying data collected by the assembly 1002 of a particular patient may adjust or otherwise control how the assembly1002 collects future data.

The amount and type of data collected by an assembly 1002 may be different for different body parts, for different types of patient conditions, for different patient demographics, or for other differences. Alternatively, or in addition, the amount and type of data collected may change overtime based on other factors, such as how the patient is healing or feeling, how long the monitoring process is projected to last, how much power remains in the assembly 1002 and should be conserved, the type of movement being monitored, the body part being monitored, and the like. In some cases, the collected data can be supplemented with personally descriptive information provided by the patient, such as subjective pain data, quality of life metric data, co-morbidities, perceptions or expectations that the patient associates with the assembly 1002, or the like.

Once the assembly 1002 is implanted into the patient and the patient returns home, the assembly 1002 may begin communicating with external devices outside of the patient's body, within the home environment. The communication may be with, e.g., the home base station 1004, the smart device 1005 (e.g., the patient's smart phone), and/or a connected personal assistant 1007, or two or more of the home base station 1004, and the smart device 1005, and the connected personal assistant 1007 can communicate with the assembly 1002. The assembly 1002 can collect data at determined rates and times, variable rates and times, or otherwise controllable rates and times. Data collection can start when the assembly 1002 is initialized in the operating room, when directed by a medical practitioner, or at some later point in time. At least some data collected by the assembly 1002 may be transmitted directly to the home base station 1004, the smart device 1005, and/or the connected personal assistant 1007. At least some data collected by the assembly 1002 can be transmitted indirectly to home base station 1004, the smart device 1005, and/or the personal assistant 1007. For example, the data can be transmitted to the base station 1004 via the smart device 1005 and/or the personal assistant 1007, to the smart device 1005 via the base station 1004 and/or the connected personal assistant 1007, or to the connected personal assistant 1007 via the smart device 1005 and/or the base station 1004. Here, "and/or" means via an item alone, and via both items serially or in parallel. For example, data collected by the assembly 1002 may be transmitted to the home base station 1004 via the smart device 1005 alone, via the connected personal assistant 1007 alone, serially via the smart device 1005 and the connected personal assistant 1007, serially via the connected personal assistant 1007 and the smart device 1005, and directly, and possibly contemporaneously, via both the smart device 1005 and the connected personal assistant 1007. Similarly, data collected by the assembly 1002 may be transmitted to the smart device 1005 via the home base station 1004 alone, via the connected personal assistant 1007 alone, serially via the home base station 1004 and the connected personal assistant 1007, serially via the connected personal assistant 1007 and the home base station 1004, and directly, and possibly contemporaneously, via both the home base station 1004 and the connected personal assistant 1007. Further in example, data collected by the assembly 1002 may be transmitted to the connected personal assistant 1007 via the smart device 1005 alone, via the home base station 1004 alone, serially via the smart device 1005 and the home base station 1004, serially via the home base station 1004 and the smart device 1005, and directly, and possibly contemporaneously, via both the smart device 1005 and the home base station 1004.

In various embodiments, one or more of the home base station 1004, the smart device 1005, and the connected personal assistant 1007 can ping the assembly 1002 at periodic, predetermined, or other times to determine if the assembly 1002 is within communication range of one or more of the home base station 1004, the smart device 1005, and the connected personal assistant 1007. Based on a response from the assembly 1002, one or more of the home base station 1004, the smart device 1005, and the connected personal assistant 1007 can determine that the assembly 1002 is within communication range, and the assembly 1002 can be requested, commanded, or otherwise directed to transmit the data it has collected to one or more of the home base station 1004, the smart device 1005, and the connected personal assistant 1007.

Each of one or more of the home base station 1004, the smart device 1005, and the connected personal assistant 1007 may, in some cases, be arranged with a respective optional user interface. The user interface may be formed as a multimedia interface that unidirectionally or bi-directionally passes one or more types of multimedia information (e.g., video, audio, tactile, etc.). Via the respective user interface of one or more of the home base station 1004, the smart device 1005, and the connected personal assistant 1007, the patient (not shown in Fig. 3) or an associate (not shown in Fig. 3) of the patient may enter other data to supplement the data collected by the assembly 1002. A user, for example, may enter personally descriptive information (e.g., age change, weight change), changes in medical condition, co-morbidities, pain levels, quality of life, an indication of how the implanted device 1002 "feels," or other subjective metric data, personal messages for a medical practitioner, and the like. In these embodiments, the personally descriptive information may be entered with a keyboard, mouse, touch-screen, microphone, wired or wireless computing interface, or some other input means. In cases where the personally descriptive information is collected, the personally descriptive information may include, or otherwise be associated with, one or more identifiers that associate the information with unique identifier of the assembly 1002, the patient, an associated medical practitioner, an associated medical facility, or the like.

In some of these cases, a respective optional user interface of each of one or more of the home base station 1004, the smart device 1005, and the connected personal assistant 1007 may also be arranged to deliver information associated with the assembly 1002 to the user from, for example, a medical practitioner. In these cases, the information delivered to the user may be delivered via a video screen, an audio output device, a tactile transducer, a wired or wireless computing interface, or some other like means.

In embodiments where one or more of the home base station 1004, the smart device 1005, and the connected personal assistant 1007 are arranged with a user interface, which may be formed with an internal user interface arranged for communicative coupling to a patient portal device. The patient portal device may be a smartphone, a tablet, a body-worn device, a weight or other health measurement device (e.g., thermometer, bathroom scale, etc.), or some other computing device capable of wired or wireless communication. In these cases, the user can enter the personally descriptive information and receive information associated with the implantable device 1002 via the internal user interface and/or the patient portal device.

The home base station 1004 can utilize a home network 1006 of the patient to transmit the collected data to the cloud 1008. The home network 1006, which may be a local area network, can provide access from the home of the patient to a wide area network, such as the internet. In some embodiments, the home base station 1004 may utilize a Wi-Fi connection to connect to the home network 1006 and access the internet. In other embodiments, the home base station 1004 may be connected to a home computer (not shown in Fig. 3) of the patient, such as via a USB connection, which itself is connected to the home network 1006.

The smart device 1005 can communicate with the assembly 1002 directly via, for example, Bluetooth^{™} compatible signals, and can utilize the home network 1006 of the patient to transmit the collected data to the cloud 1008, or can communicate directly with the cloud 1008, for example, via a cellular network. In some configurations, the smart device 1005 can be configured to communicate directly with one or both of the home base station 1004 and the connected personal assistant 1007 via, for example, Bluetooth^{™} compatible signals, and may not be configured to communicate directly with the assembly 1002.

Furthermore, the connected personal assistant 1007 can communicate with the assembly 1002 directly via, for example, Bluetooth^{™} compatible signals, and can utilize the home network 1006 of the patient to transmit the collected data to the cloud 1008, or can communicate directly with the cloud 1008 (e.g., via a modem/internet connection or a cellular network). In some configurations, the connected personal assistant 1007 can be configured to communicate directly with one or both of the home base station 1004 and the smart device 1005 via, for example, Blue Tooth^{®} compatible signals, and may not configured to communicate directly with the assembly 1002.

Along with transmitting collected data to the cloud 1008, one or more of the home base station 1004, the smart device 1005, and the connected personal assistant 1007 may also obtain data, commands, or other information from the cloud 1008 directly or via the home network 1006. One or more of the home base station 1004, the smart device 1005, and the connected personal assistant 1007 may provide some or all of the received data, commands, or other information to the assembly 1002. Examples of such information include, but are not limited to, updated configuration information, diagnostic requests to determine if the assembly 1002 is functioning properly, data collection requests, and other information.

The cloud 1008 may include one or more server computers or databases to aggregate data collected from the assembly 1002, and in some cases personally descriptive information collected from the patient (not shown in Fig. 3), with data collected from other assemblies (not illustrated), and in some cases personally descriptive information collected from other patients. In this way, the cloud 1008 can create a variety of different metrics regarding collected data from each of a plurality of assemblies 1002 that are implanted into separate patients. This information can be helpful in determining if the assemblies 1002 are functioning properly. The collected information may also be helpful for other purposes, such as determining which specific devices 1002 may not be functioning properly, determining if a procedure or condition associated with the assembly 1002 is helping the patient (e.g., if the knee replacement is operating properly and reducing the patient's pain), and determining other medical information.

In some configurations, one or two of the home base station 1004, the smart device 1005, and the connected personal assistant 1007 may be omitted from the assembly environment 1000. In some configurations, each of the home base station 1004, the smart device 1005, and the connected personal assistant 1007 may be configured to communicate with one or both of the implantable device 1002 and the cloud 1008 via another the base station 1004, the smart device 1005, and/or the connected personal assistant 1007. In some configurations, the smart device 1005 can be used as an interface to the implantable device 1002. The smart device 1005 can be any suitable device other than a smart phone, such as a smart watch, a smart patch, and any loT device (e.g., a coffee pot) capable of acting as an interface to the implantable device 1002. In some configurations, one or more of the home base station 1004, smart device 1005, and connected personal assistant 1007 can act as a communication hub for multiple prostheses and/or assemblies 1002 implanted in one or more patients. In some configurations, one or more of the home base station 1004, the smart device 1005, and the connected personal assistant 1007 can be configured to automatically order or reorder prescriptions or medical supplies (e.g., a knee brace) in response to patient input or implantable-prosthesis input (e.g., pain level, instability level) if a medical professional and insurance company have preauthorized such an order or reorder. In some configurations, one or more of the base station 1004, the smart device 1005, and the connected personal assistant 1007 can be configured to request, from a medical professional or an insurance company, authorization to place the order or reorder. In some configurations, one or more of the home base station 1004, the smart device 1005, and the connected personal assistant 1007 can be configured with a personal assistant such as Alexa^{®} or Siri^{®}. Such a personal assistant is advantageous to a patient in that it provides access to a conversational artificial intelligence (AI) and/or an interactive patient experience. Such a personal assistant may be particularly useful for a patient having a physical or mental impairment, as can sometimes, even often, come with aging of the patient. Such a personal assistant provides a useful alternative to communication by the patient via a smartphone, where some patients have physical or mental limitations that make operating a smart phone challenging to the point of being a non-useful tool. Such a personal assistant provides the patient with access to the resources available to the personal assistant. For example, Alexa^{®} has access to the product sales infrastructure that has been created by the Amazon company, such many patients would benefit by such access to secure products, for example, pharmaceuticals or devices such as a walker or cane.

In one embodiment, a patient secures a wearable monitor to themselves. The wearable monitor may provide the patient with access to a personal assistant such as Alexa^{®} or Siri^{®}, where the access is optionally via a smart display such as an Echo Show by Amazon. The personal assistant may do one or more of identify and authenticate the patient, interact with the patient to obtain subjective patient data (e.g., the personal assistant may question the patient about how he or she is feeling today and then store the patient's reply), provides results to date, and/or offer the patient useful links or other assistance as requested by the patient. As some patients may not be too savvy with using a personal assistant or may have some physical or mental impairment that makes interacting with a personal assistant challenging for the patient, in one embodiment the user interface is simplistic and easy for the patient to interact with. For example, the present disclosure provides that identification and authentication of the patient may be done by means other than speech, such as facial recognition, or voice recognition.

The personal assistant may optionally facilitate a video conference with an attending physician. In addition to facilitating the implementation of a video conference, the personal assistant may assist in communication between the attending health care provider (HCP) and the patient, e.g., by repeating, at a louder volume, the questions and/or requests of the health care provider during the video conference. During the video conference, the HCP may request that the patient walk around in a manner that allows the HCP to observe the movement in real time. The personal assistant may assist in directing the camera to the patient during this movement.

The personal assistant may optionally assist the patient with access to a pharmaceutical supply chain so that desired medicaments can be easily obtained by the patient. The personal assistant may optionally assist the patient with access to pre-stocked demo user history, including live user results. The personal assistant may actively ask the patient if certain information would be useful, e.g., the personal assistant may ask the patient is he or she would like to view videos of exercises that might be helpfully performed by the patient. The personal assistant can record the answer and facilitate access to videos as appropriate.

Ready access to a personal assistant as provided by the present disclosure can allow the patient to secure appointments with a health care provider (HCP) or conduct a teleconference with their HCP. Ready access to a personal assistant and a conveniently located monitor provides the patient with access to videos that may be viewed and the information therefrom used to enhance the patient's quality of life, particularly as it may relate to a medical condition and overcoming any limitations caused by the presence of the medical condition. Ready access to a personal assistant as provided by the present disclosure may be useful to the patient in readily securing new or additional dosages of medication that is being utilized by the patient, or perhaps suitable alternatives to such medication.

Ready access to a personal assistant as provided by the present disclosure may be useful to the patient in accessing suitable social media, where the patient may learn from, and/or interact with, other people who share similar interests, e.g., have similar medical conditions. For example, the personal assistant may facilitate communication between the patient and social media accessed by friends and family of the patient, and may even post information about the patient to such social media for the benefit of the friends and family, so that, for example, the friends and family are appraised of the healing progress of the patient. Ready access to a personal assistant as provided by the present disclosure may be useful to the patient in accessing written or visual information located on the internet, such as suitable links to useful information. Ready access to a personal assistant as provided by the present disclosure may be useful to the patient in access emergency services, such as providing by calling 911. For example, the patient may instruct the personal assistant to call 911, and then facilitate communication between the patient and the operator that answers the 911 call, e.g., providing the address where the patient is located.

Although the assembly environment 1000 has been described in the context of a patient's home by reference to Fig. 3, the same principles apply when the environment is an operating room or a doctor's office. For example, in association with a medical procedure, an assembly 1002 may be implanted in the patient's body within an operating room environment. Coetaneous with the medical procedure, the assembly 1002 can communicate with an operating room base station (analogous to the home base station 1004). Subsequently, after sufficient recovery from the medical procedure, the patient can return home and the assembly 1002 can be arranged to communicate with a home base station 1004. Thereafter, at other times, the assembly 1002 can be arranged to communicate with a doctor office base station when the patient visits the doctor for a follow-up consultation. In any case, the assembly 1002 can communicate with each base station via a short range network protocol, such as the medical implant communication service (MICS), the medical device radio communications service (MedRadio), or some other wireless communication protocol suitable for use with the assembly 1002.

For example, implantation of the assembly 1002 into the patient may occur in an operating room. As used herein, operating room includes any office, room, building, or facility where the assembly 1002 is implanted into the patient. For example, the operating room may be a typical operating room in a hospital, an operating room in a surgical clinic or a doctor's office, or any other operating theater where the assembly 1002 is implanted into the patient.

The operating room base station (analogous to the home base station 1004 of Fig. 3) can be utilized to configure and initialize the assembly 1002 in association with the assembly 1002 being implanted into the patient. A communicative relationship can be formed between the assembly 1002 and the operating room base station, for example, based on a polling signal transmitted by the operating room base station and a response signal transmitted by the assembly 1002.

Upon forming a communicative relationship, which can occur prior to implantation of the assembly 1002, the operating room base station can transmit initial configuration information to the assembly 1002. This initial configuration information may include, but is not limited to, a time stamp, a day stamp, an identification of the type and placement of the assembly 1002, information on other implants associated with the assembly, surgeon information, patient identification, operating room information, and the like.

In some embodiments, the initial configuration information can be passed unidirectionally or bidirectionally. The initial configuration information may define at least one parameter associated with the collection of data by the assembly 1002. For example, the configuration information may identify settings for one or more sensors on the assembly 1002 for each of one or more modes of operation. The configuration information may include other control information, such as an initial mode of operation of the assembly 1002, a particular event that triggers a change in the mode of operation, radio settings, data collection information (e.g., how often the assembly 1002 wakes up to collected data, how long it collects data, how much data to collect), home base station 1004, smart device 1005, and connected personal assistant 1007 identification information, and other control information associated with the implantation or operation of the assembly 1002. Examples of the connected personal assistant 1007, which also can be called a smart speaker, include Amazon Echo^{®}, Amazon Dot^{®}, Google Home^{®}, Philips^{®} patient monitor, Comcast's health-tracking speaker, and Apple HomePod^{®}.

In some embodiments, the configuration information may be pre-stored on the operating room base station or an associated computing device. In some embodiments, a surgeon, surgical technician, or some other medical practitioner may input the control information and other parameters to the operating room base station for transmission to the assembly 1002. In at least one such embodiment, the operating room base station may communicate with an operating room configuration computing device. The operating room configuration computing device can include an application with a graphical user interface that enables the medical practitioner to input configuration information for the assembly 1002. In various embodiments, the application executing on the operating room configuration computing device may have some of the configuration information predefined, which may or may not be adjustable by the medical practitioner.

The operating room configuration computing device can communicate the configuration information to the operating room base station via a wired or wireless network connection (e.g., via a USB connection, Bluetooth^{™} connection, Bluetooth^{™} Low Energy (BTLE) connection, or Wi-Fi connection). The operating room base station can communicate the configuration information to the assembly 1002.

The operating room configuration computing device may display information regarding the assembly 1002 or the operating room base station to the surgeon, surgical technician, or other medical practitioner. For example, the operating room configuration computing device may display error information if the assembly 1002 is unable to store or access the configuration information, if the assembly 1002 is unresponsive, if the assembly 1002 identifies an issue with one of the sensors or radio during an initial self-test, if the operating room base station is unresponsive or malfunctions, or for other reasons.

Although the operating room base station and the operating room configuration computing device are described as separate devices, embodiments are not so limited; rather, the functionality of the operating room configuration computing device and the operating room base station may be included in a single computing device or in separate devices as illustrated. In this way, the medical practitioner may be enabled in one embodiment to input the configuration information directly into the operating room base station.

After the assembly 1002 has been implanted in the patient, the patient may periodically visit a doctor's office for follow-up evaluation. In one aspect, the present disclosure provides a doctor's office environment that can be analogous to the home environment 1000. For example, the implanted assembly 1000 can communicate with the office environment. During these visits, the data that has been stored in memory of the assembly 1000 may be accessed, and/or specific data may be requested and obtained as part of a monitoring process.

For example, at various times throughout the monitoring process, the patient may be requested to visit a medical practitioner for follow up appointments. This medical practitioner may be the surgeon who implanted the assembly 1002 in the patient or a different medical practitioner that supervises the monitoring process, physical therapy, and/or recovery of the patient. For a variety of different reasons, the medical practitioner may want to collect real-time data from the assembly 1002 in a controlled environment. In some cases, the request to visit the medical practitioner may be delivered through a respective optional bidirectional user interface of each of one or more of the home base station 1004, the smart device 1005, and the connected personal assistant 1007.

A medical practitioner can utilize the doctor office base station (analogous to the home base station 1004 shown in Fig. 3), which can communicate with the assembly 1002, to pass additional data between the doctor office base station and the assembly 1002. Alternatively, or in addition, the medical practitioner can utilize the doctor office base station (not shown in Fig. 3) to pass commands to the assembly 1002. In some embodiments, the doctor office base station can instruct the assembly 1002 to enter a high-resolution mode to temporarily increase the rate or type of data that is collected for a short time. The high-resolution mode can direct the assembly 1002 to collect different (e.g., large) amounts of data during an activity where the medical practitioner is also monitoring the patient.

In some embodiments, the doctor office base station can enable the medical practitioner to input event or pain markers, which can be synchronized with the high-resolution data collected by the assembly 1002. For example, the medical practitioner can have the patient walk on a treadmill while the assembly 1002 is in the high-resolution mode. As the patient walks, the patient may complain about pain. The medical practitioner can click a pain marker button on the doctor office base station to indicate the patient's discomfort. The doctor office base station can records the marker and the time at which the marker was input. The timing of this marker can be synchronized with the timing of the collected high-resolution data such that the medical practitioner can analyze the data to try to determine the cause of the pain.

In some embodiments, the doctor office base station may provide updated configuration information to the assembly 1002. The assembly 1002 can store this updated configuration information, which can be used to adjust the parameters associated with the collection of the data. For example, if the patient is doing well, the medical practitioner can direct a reduction in the frequency at which the assembly 1002 collects data. On the contrary, if the patient is experiencing an unexpected amount of pain, the medical practitioner may direct the assembly 1002 to collect additional data for a determined period of time (e.g., a few days). The medical practitioner may use the additional data to diagnose and treat a particular problem. In some cases, the additional data may include personally descriptive information provided by the patient after the patient has left presence of the medical practitioner and is no longer in range of the doctor office base station. In these cases, the personally descriptive information may be collected and delivered from via one or more of the home base station 1004, the smart device 1005, and the connected personal assistant 1007. Firmware within the assembly 1002 and/or the base station 1004 can provide safeguards limiting the duration of such enhanced monitoring to ensure the assembly 1002 retains sufficient power to last for the implant's lifecycle.

In various embodiments, the doctor office base station may communicate with a doctor office configuration computing device, which can be analogous to the operating room computing device. The doctor office configuration computing device can include an application with a graphical user interface that can receive commands and data from the medical practitioner. Some or all of the commands, data, and other information may be later transmitted to the assembly 1002 via the doctor office base station. For example, in some embodiments, the medical practitioner can use the graphical user interface to instruct the assembly 1002 to enter its high-resolution mode. In some embodiments, the medical practitioner can use graphical user interface to input or modify the configuration information for the assembly 1002. The doctor office configuration computing device can transmit the information (e.g., commands, data, or other information) to the doctor office base station via a wired or wireless network connection (e.g., via a USB connection, Bluetooth^{™} connection, or Wi-Fi connection), which in turn can transmit some or all of the information to the assembly 1002.

The doctor office configuration computing device may display other information regarding the assembly 1002, regarding the patient (e.g., personally descriptive information), and/or the doctor office base station to the medical practitioner. For example, the doctor office configuration computing device may display the high-resolution data that is collected by the assembly 1002 and transmitted to the doctor office base station. The doctor office configuration computing device may display error information if the assembly 1002 is unable to store or access the configuration information, if the assembly 1002 is unresponsive, if the assembly 1002 identifies an issue with one of the sensors or radio, if the doctor office base station is unresponsive or malfunctions, or for other reasons.

In some embodiments, doctor office configuration computing device may have access to the cloud 1008. In some embodiments, the medical practitioner can utilize the doctor office configuration computing device to access data stored in the cloud 1008, which was previously collected by the assembly 1002 and transmitted to the cloud 1008 via one or both of the home base station 1004 and smart device 1005. Similarly, the doctor office configuration computing device can transmit the high-resolution data obtain from the assembly 1002 via the doctor office base station to the cloud 1008. In some embodiments, the doctor office base station may have internet access and may be enabled to transmit the high-resolution data directly to the cloud 1008 without the use of the doctor office configuration computing device.

In various embodiments, the medical practitioner may update the configuration information of the assembly 1002 when the patient is not in the medical practitioner's office. In these cases, the medical practitioner can utilize the doctor office configuration computing device (not shown in Fig. 3) to transmit updated configuration information to the assembly 1002 via the cloud 1008. One or more of the home base station 1004, the smart device 1005, and the connected personal assistant 1007 can obtain updated configuration information from the cloud 1008 and pass updated configuration information to the cloud 1008. This can allow the medical practitioner to remotely adjust the operation of the assembly 1002 without needing the patient to come to the medical practitioner's office. This may also permit the medical practitioner to send messages to the patient in response, for example, to personally descriptive information that was provided by the patient and passed through one or more of the home base station 1004, the smart device 1005, and the connected personal assistant 1007 to the doctor office base station. For example, if a patient speaks "I feel pain" into the connected personal assistant 1007, the medical practitioner may issue a prescription for a pain reliever and can cause the connected personal assistant 1007 to notify the patient by "speaking" "the doctor has called in a prescription for Vicodin^{®} to your preferred pharmacy; the prescription will be ready for pick up at 4pm."

Although the doctor office base station (not shown in Fig. 3) and the doctor office configuration computing device (not shown in Fig. 3) are described as separate devices, embodiments are not so limited; rather, the functionality of the doctor office configuration computing device and the doctor office base station may be included in a single computing device or in separate devices (as illustrated). In this way, the medical practitioner may be enabled in one embodiment to input the configuration information or markers directly into the doctor office base station and view the high-resolution data and any synchronized marker information from a display on the doctor office base station.

### VIII. ADDITIONAL EXEMPLARY SPECIFIC EMBODIMENTS

In one aspect, the present disclosure provides an assembly that obtains information about the pressure and/or vibration present in the vessel where the assembly is implanted.

In one aspect, the assembly of the present disclosure may be implanted in the cardiovascular system of a patient. As used herein, the cardiovascular system of a patient refers to the circulatory system which comprises the heart and blood vessels and carries nutrients and oxygen to the tissues of the body and removes carbon dioxide and other wastes from them. In one aspect, the assembly of the present disclosure may be implanted in an artery of a patient. In one aspect, the assembly may be implanted in any artery of a patient where information about the state of that artery is desired. In one aspect, the assembly of the present disclosure may be implanted in a coronary artery of a patient. In one aspect, the assembly of the present disclosure may be implanted in a vein of a patient. In one aspect, the assembly of the present disclosure may be implanted in a vein at a location where information about the state of that vein is desired.

In one aspect, the assembly of the present disclosure is implanted in the vicinity of a stent which has likewise been implanted in a body passageway of the patient. The assembly of the present disclosure may be implanted prior to, essentially simultaneously with (i.e., during the same medical procedure), or after, the implantation of the stent.

In one aspect, the present disclosure provides a system, where the system comprises an assembly of the present disclosure along with one or more auxiliary items such as (i) a base station that receives information from the assembly (particularly an implanted assembly) via wireless communication between the assembly and the base station, (ii) a barcode scanner that can scans a barcode that identifies the assembly and optionally associates that identification with specific details pertinent to the implantation of the specific assembly, e.g., details about the patient and/or the procedure by which the assembly is implanted, (iii) a charger for the power source, e.g., a charger that can achieve inductive charging of the power source that has been implanted in the patient. The auxiliary item may be in communication with (e.g., via a USB port, or via wireless communication, as two examples) a computer, e.g., a laptop. Thus, the system may include a computer.

The assembly of the present disclosure may be used in conjunction with the treatment of a vessel lesion, such as a coronary lesion. The assembly of the present disclosure may comprise a pressure sensor. The assembly comprising a pressure sensor may be used in conjunction with the treatment of a vessel lesion, such as a coronary lesion.

It is also to be understood that as used herein and in the appended claims, the singular forms "a," "an," and "the" include plural reference unless the context clearly dictates otherwise, the term "X and/or Y" means "X" or "Y" or both "X" and "Y", and the letter "s" following a noun designates both the plural and singular forms of that noun. In addition, where features or aspects of the embodiments are described in terms of Markush groups, it is intended, and those skilled in the art will recognize, that the embodiments embraces and is also thereby described in terms of any individual member and any subgroup of members of the Markush group, and Applicants reserve the right to revise the application or claims to refer specifically to any individual member or any subgroup of members of the Markush group.

It is to be understood that the terminology used herein is for the purpose of describing specific embodiments only and is not intended to be limiting. It is further to be understood that unless specifically defined herein, the terminology used herein is to be given its traditional meaning as known in the relevant art.

Reference throughout this specification to "one embodiment" or "an embodiment" and variations thereof means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used in this specification and the appended claims, the singular forms "a," "an," and "the" include plural referents, i.e., one or more, unless the content and context clearly dictates otherwise. It should also be noted that the conjunctive terms, "and" and "or" are generally employed in the broadest sense to include "and/or" unless the content and context clearly dictates inclusivity or exclusivity as the case may be. Thus, the use of the alternative (e.g., "or") should be understood to mean either one, both, or any combination thereof of the alternatives. In addition, the composition of "and" and "or" when recited herein as "and/or" is intended to encompass an embodiment that includes all of the associated items or ideas and one or more other alternative embodiments that include fewer than all of the associated items or ideas.

Unless the context requires otherwise, throughout the specification and claims that follow, the word "comprise" and synonyms and variants thereof such as "have" and "include", as well as variations thereof such as "comprises" and "comprising" are to be construed in an open, inclusive sense, e.g., "including, but not limited to." The term "consisting essentially of" limits the scope of a claim to the specified materials or steps, or to those that do not materially affect the basic and novel characteristics of the claimed embodiments.

Where a range of values is provided herein, it is understood that each intervening value, to the tenth of the unit of the lower limit unless the context clearly dictates otherwise, between the upper and lower limit of that range and any other stated or intervening value in that stated range is encompassed within the embodiments. The upper and lower limits of these smaller ranges may independently be included in the smaller ranges is also encompassed within the embodiments, subject to any specifically excluded limit in the stated range. Where the stated range includes one or both of the limits, ranges excluding either or both of those included limits are also included in the embodiments.

For example, any concentration range, percentage range, ratio range, or integer range provided herein is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated. As used herein, the term "about" means ± 20% of the indicated range, value, or structure, unless otherwise indicated.

In general, in the following claims, the terms used should not be construed to limit the claims to the specific embodiments disclosed in the specification and the claims, but should be construed to include all possible embodiments along with the full scope of equivalents to which such claims are entitled.

Other nonlimiting embodiments are within the following claims. The patent may not be interpreted to be limited to the specific examples or nonlimiting embodiments or methods specifically and/or expressly disclosed herein.

## Claims

1. An implantable sensor assembly (10) comprising:
a first anchor (15) and a second anchor (15), the first and second anchors (15) configured to maintain a position of the implantable sensor assembly (10) in a body passageway of a patient, the first anchor (15) connected to the second anchor (15); wherein the first anchor (15) and the second anchor (215) are a tacking stent;
a sensor system (25) comprising a first sensor and a second sensor, wherein the first sensor is directly coupled to the first anchor (15) and the second sensor is directly coupled to the second anchor (15), the sensor system (25) being configured to collect sensor data related to one or more characteristics of the body passageway of the patient; and
a communications circuitry (35) configured to wirelessly communicate with one or more external devices, wherein the communications circuitry (35) extends from the first sensor to the second sensor, or wherein the communications circuitry (35) extends from the first anchor (15) to the second anchor (15).

2. The implantable sensor assembly (10) of Claim 1, wherein the communications circuitry (35) comprises an antenna.

3. The implantable sensor assembly (10) of Claim 1, wherein the communications circuitry (35) comprises a wakeup receiver configured to detect a wakeup signal from the one or more external devices and to activate the sensor assembly (10) in response to detecting the wakeup signal.

4. The implantable sensor assembly (10) of Claim 1, wherein at least one of the first sensor or the second sensor is a blood flow sensor, a blood pressure sensor, a metabolic sensor, a glucose sensor, a pressure sensor, an oxygen sensor, or a protein enzyme sensor.

5. The implantable sensor assembly (10) of Claim 1, wherein each of the first and second anchors (15) is configured to expand from a first diameter in a delivery configuration to a second diameter in a deployed configuration; or
wherein each of the first and second anchors has a length less than or equal to about 9 mm.

6. The implantable sensor assembly (10) of Claim 1, wherein at least one of the first anchor (15) or second anchor (15) comprises a plurality of struts and a plurality of cells between the plurality of struts, preferably
wherein at least one cell of the plurality of cells is sized and configured to receive the sensor system (25), preferably
wherein the sensor system (25) is configured to be coupled to a crown of the plurality of struts; or
wherein the sensor system is configured to be coupled to an edge of the first anchor or the second anchor.

7. The implantable sensor assembly (10) of Claim 1, wherein the communications circuitry (35) is configured to wirelessly transmit raw data collected from the sensor system (25).

8. The implantable sensor assembly (10) of Claim 1, wherein the sensor system comprises processing circuitry configured to at least partially process the sensor data collected from the first sensor and the second sensor, preferably
wherein the communications circuitry (35) is configured to wirelessly transmit the at least partially processed sensor data; or wherein the communications circuitry (35) is configured to wirelessly receive instructions from the one or more external devices; or
wherein the implantable sensor assembly (10) is configured to receive power from the one or more external devices.

9. The implantable sensor assembly (10) of Claim 1, further comprising a power source (30) configured to provide power to the sensor assembly, preferably
wherein the power source (30) is rechargeable, preferably
wherein the power source (30) is configured to receive power from the one or more external devices, preferably
wherein the power source (30) comprises a battery or a capacitor; or
wherein the power source (30) is hermetically sealed; or wherein the sensor assembly (10) is configured to be powered by a power source (30) outside the patient.

10. The implantable sensor assembly (10) of Claim 1, wherein the one or more characteristics comprises pressure, flow, sound, vibration, or appearance of the environment surrounding the implantable sensor assembly; or
wherein the sensor system (25) is hermetically sealed.

11. The implantable sensor assembly (10) of Claim 1, further comprising a unique identification code comprising information about the implantable sensor assembly (10), preferably
wherein the unique identification code is configured to be scanned by a barcode scanner; or
wherein the unique identification code is integrated with a RFID.

12. The implantable sensor assembly (10) of Claim 1, further comprising a memory device for storing the sensor data related to the one or more characteristics.

13. The implantable sensor assembly (10) of Claim 1, wherein the communications circuitry (35) is configured to wirelessly communicate with the one or more external devices via a Bluetooth^{™} protocol, WiFi, ZigBee, medical implant communication service ("MICS"), the medical device radio communications service ("MedRadio"), or cellular telephony.

14. A kit comprising:
the implantable sensor assembly (10) of Claim 1; and
a delivery system (45) configured to deliver the sensor assembly to the body passageway of the patient, preferably
wherein the delivery system (45) is a balloon catheter; or
wherein the delivery system (45) comprises a sheath configured to cover the first and second anchors (15) when delivering the implantable sensor assembly to the body passageway of the patient.

## Patentansprüche

1. Implantierbare Sensoranordnung (10), umfassend:
einen ersten Anker (15) und einen zweiten Anker (15), wobei der erste und der zweite Anker (15) konfiguriert sind, um eine Position der implantierbaren Sensoranordnung (10) in einem Körperkanal eines Patienten beizubehalten, wobei der erste Anker (15) mit dem zweiten Anker (15) verbunden ist; wobei der erste Anker (15) und der zweite Anker (215) ein Verankerungsstent sind;
ein Sensorsystem (25), umfassend einen ersten Sensor und einen zweiten Sensor, wobei der erste Sensor direkt an den ersten Anker (15) gekoppelt ist und der zweite Sensor direkt an den zweiten Anker (15) gekoppelt ist, wobei das Sensorsystem (25) konfiguriert ist, um Sensordaten zu erfassen, die sich auf eine oder mehrere Eigenschaften des Körperkanals des Patienten beziehen; und
eine Kommunikationsschaltung (35), die konfiguriert ist, um drahtlos mit einer oder mehreren externen Vorrichtungen zu kommunizieren, wobei sich die Kommunikationsschaltung (35) von dem ersten Sensor zu dem zweiten Sensor erstreckt, oder wobei sich die Kommunikationsschaltung (35) von dem ersten Anker (15) zu dem zweiten Anker (15) erstreckt.

2. Implantierbare Sensoranordnung (10) nach Anspruch 1, wobei die Kommunikationsschaltung (35) eine Antenne umfasst.

3. Implantierbare Sensoranordnung (10) nach Anspruch 1, wobei die Kommunikationsschaltung (35) einen Aufweckempfänger umfasst, der konfiguriert ist, um ein Aufwecksignal von der einen oder den mehreren externen Vorrichtungen zu erfassen und die Sensoranordnung (10) als Reaktion auf das Erfassen des Aufwecksignals zu aktivieren.

4. Implantierbare Sensoranordnung (10) nach Anspruch 1, wobei mindestens einer des ersten Sensors oder des zweiten Sensors ein Blutflusssensor, ein Blutdrucksensor, ein Stoffwechselsensor, ein Glukosesensor, ein Drucksensor, ein Sauerstoffsensor oder ein Proteinenzymsensor ist.

5. Implantierbare Sensoranordnung (10) nach Anspruch 1, wobei jeder der ersten und zweiten Anker (15) konfiguriert ist, um sich von einem ersten Durchmesser in einer Zuführkonfiguration auf einen zweiten Durchmesser in einer Anwendungskonfiguration auszudehnen; oder
wobei jeder des ersten und des zweiten Ankers eine Länge von weniger als oder gleich etwa 9 mm aufweist.

6. Implantierbare Sensoranordnung (10) nach Anspruch 1, wobei mindestens eines von dem ersten Anker (15) oder dem zweiten Anker (15) eine Vielzahl von Streben und eine Vielzahl von Zellen zwischen der Vielzahl von Streben umfasst, wobei
vorzugsweise mindestens eine Zelle der Vielzahl von Zellen bemessen und konfiguriert ist, um das Sensorsystem (25) aufzunehmen, wobei
vorzugsweise das Sensorsystem (25) konfiguriert ist, um an eine Krone der Vielzahl von Streben gekoppelt zu werden; oder
wobei das Sensorsystem konfiguriert ist, um an eine Kante des ersten Ankers oder des zweiten Ankers gekoppelt zu werden.

7. Implantierbare Sensoranordnung (10) nach Anspruch 1, wobei die Kommunikationsschaltung (35) konfiguriert ist, um vom Sensorsystem (25) gesammelte Rohdaten drahtlos zu übertragen.

8. Implantierbare Sensoranordnung (10) nach Anspruch 1, wobei das Sensorsystem eine Verarbeitungsschaltung umfasst, die konfiguriert ist, um die vom ersten Sensor und vom zweiten Sensor erfassten Sensordaten mindestens teilweise zu verarbeiten, wobei
vorzugsweise die Kommunikationsschaltung (35) konfiguriert ist, um die mindestens teilweise verarbeiteten Sensordaten drahtlos zu übertragen; oder wobei die Kommunikationsschaltung (35) konfiguriert ist, um drahtlos Anweisungen von der einen oder den mehreren externen Vorrichtungen zu empfangen; oder
wobei die implantierbare Sensoranordnung (10) konfiguriert ist, um Energie von der einen oder den mehreren externen Vorrichtungen zu empfangen.

9. Implantierbare Sensoranordnung (10) nach Anspruch 1, ferner umfassend eine Energiequelle (30), die konfiguriert ist, um der Sensoranordnung Energie bereitzustellen, wobei
vorzugsweise die Energiequelle (30) wiederaufladbar ist, wobei
vorzugsweise die Energiequelle (30) konfiguriert ist, um Energie von der einen oder den mehreren externen Vorrichtungen zu empfangen, wobei
vorzugsweise die Energiequelle (30) eine Batterie oder einen Kondensator umfasst; oder
wobei die Energiequelle (30) hermetisch abgedichtet ist; oder wobei die Sensoranordnung (10) konfiguriert ist, um von einer Energiequelle (30) außerhalb des Patienten mit Energie versorgt zu werden.

10. Implantierbare Sensoranordnung (10) nach Anspruch 1, wobei die eine oder mehreren Eigenschaften Druck, Durchfluss, Schall, Vibration oder Erscheinungsbild der die implantierbare Sensoranordnung umgebenden Umgebung umfassen; oder
wobei das Sensorsystem (25) hermetisch abgedichtet ist.

11. Implantierbare Sensoranordnung (10) nach Anspruch 1, ferner umfassend einen eindeutigen Identifikationscode, der Informationen über die implantierbare Sensoranordnung (10) umfasst, wobei
vorzugsweise der eindeutige Identifikationscode konfiguriert ist, um von einem Barcode-Scanner gescannt zu werden; oder
wobei der eindeutige Identifikationscode in einem RFID-Element integriert ist.

12. Implantierbare Sensoranordnung (10) nach Anspruch 1, ferner umfassend eine Speichervorrichtung zum Speichern der Sensordaten, die sich auf die eine oder mehreren Eigenschaften beziehen.

13. Implantierbare Sensoranordnung (10) nach Anspruch 1, wobei die Kommunikationsschaltung (35) konfiguriert ist, um mit der einen oder den mehreren externen Vorrichtungen über ein Bluetooth^{™}-Protokoll, WiFi, ZigBee, einen Kommunikationsdienst für medizinische Implantate ("MICS"), den Funkkommunikationsdienst für medizinische Geräte ("MedRadio") oder Mobilfunktelefonie drahtlos zu kommunizieren.

14. Kit, umfassend:
die implantierbare Sensoranordnung (10) nach Anspruch 1; und
ein Zuführsystem (45), das konfiguriert ist, um die Sensoranordnung dem Körperkanal des Patienten zuzuführen, wobei
vorzugsweise das Zuführsystem (45) ein Ballonkatheter ist; oder
wobei das Zuführsystem (45) eine Hülse umfasst, die konfiguriert ist, um den ersten und zweiten Anker (15) zu bedecken, wenn die implantierbare Sensoranordnung dem Körperkanal des Patienten zugeführt wird.

## Revendications

1. Ensemble capteur implantable (10) comprenant :
un premier ancrage (15) et un second ancrage (15), les premier et second ancrages (15) étant conçus pour maintenir une position de l'ensemble capteur implantable (10) dans un passage corporel d'un patient, le premier ancrage (15) étant relié au second ancrage (15) ; dans lequel le premier ancrage (15) et le second ancrage (215) sont une endoprothèse de fixation ;
un système de capteur (25) comprenant un premier capteur et un second capteur, dans lequel le premier capteur est directement couplé au premier ancrage (15) et le second capteur est directement couplé au second ancrage (15), le système de capteur (25) étant configuré pour collecter des données de capteur relatives à une ou plusieurs caractéristiques du passage corporel du patient ; et
un ensemble de circuits de communications (35) configuré pour communiquer sans fil avec un ou plusieurs dispositifs externes, dans lequel l'ensemble de circuits de communications (35) s'étend du premier capteur au second capteur, ou dans lequel l'ensemble de circuits de communications (35) s'étend du premier ancrage (15) au second ancrage (15).

2. Ensemble capteur implantable (10) selon la revendication 1, dans lequel l'ensemble de circuits de communications (35) comprend une antenne.

3. Ensemble capteur implantable (10) selon la revendication 1, dans lequel l'ensemble de circuits de communications (35) comprend un récepteur de réveil configuré pour détecter un signal de réveil en provenance du ou des dispositifs externes et pour activer l'ensemble capteur (10) en réponse à la détection du signal de réveil.

4. Ensemble capteur implantable (10) selon la revendication 1, dans lequel au moins l'un parmi le premier capteur ou le second capteur est un capteur de débit sanguin, un capteur de pression sanguine, un capteur métabolique, un capteur de glucose, un capteur de pression, un capteur d'oxygène ou un capteur d'enzyme protéique.

5. Ensemble capteur implantable (10) selon la revendication 1, dans lequel chacun des premier et second ancrages (15) est conçu pour s'étendre d'un premier diamètre dans une configuration de pose à un second diamètre dans une configuration déployée ; ou
dans lequel chacun des premier et second ancrages présente une longueur inférieure ou égale à environ 9 mm.

6. Ensemble capteur implantable (10) selon la revendication 1, dans lequel au moins l'un parmi le premier ancrage (15) ou le second ancrage (15) comprend une pluralité d'entretoises et une pluralité de cellules entre la pluralité d'entretoises, de préférence
dans lequel au moins une cellule de la pluralité de cellules est dimensionnée et conçue pour recevoir le système de capteur (25), de préférence
dans lequel le système de capteur (25) est configuré pour être couplé à une couronne de la pluralité d'entretoises ; ou
dans lequel le système de capteur est configuré pour être couplé à un bord du premier ancrage ou du second ancrage.

7. Ensemble capteur implantable (10) selon la revendication 1, dans lequel l'ensemble de circuits de communications (35) est configuré pour transmettre sans fil des données brutes collectées à partir du système de capteur (25).

8. Ensemble capteur implantable (10) selon la revendication 1, dans lequel le système de capteur comprend l'ensemble de circuits de traitement configuré pour traiter au moins partiellement les données de capteur collectées à partir du premier capteur et du second capteur, de préférence
dans lequel l'ensemble de circuits de communications (35) est configuré pour transmettre sans fil les données de capteur au moins partiellement traitées ; ou dans lequel l'ensemble de circuits de communications (35) est configuré pour recevoir sans fil des instructions en provenance du ou des dispositifs externes ; ou
dans lequel l'ensemble capteur implantable (10) est configuré pour recevoir de l'énergie du ou des dispositifs externes.

9. Ensemble capteur implantable (10) selon la revendication 1, comprenant en outre une source d'alimentation (30) configurée pour fournir de l'énergie à l'ensemble capteur, de préférence
dans lequel la source d'alimentation (30) est rechargeable, de préférence
dans lequel la source d'alimentation (30) est configurée pour recevoir de l'énergie du ou des dispositifs externes, de préférence
dans lequel la source d'alimentation (30) comprend une batterie ou un condensateur ; ou
dans lequel la source d'alimentation (30) est scellée hermétiquement ; ou dans lequel l'ensemble capteur (10) est configuré pour être alimenté par une source d'alimentation (30) à l'extérieur du patient.

10. Ensemble capteur implantable (10) selon la revendication 1, dans lequel la ou les caractéristiques comprennent la pression, le débit, le son, la vibration ou l'apparence de l'environnement entourant l'ensemble capteur implantable ; ou
dans lequel le système de capteur (25) est scellé hermétiquement.

11. Ensemble capteur implantable (10) selon la revendication 1, comprenant en outre un code d'identification unique comprenant des informations concernant l'ensemble capteur implantable (10), de préférence
dans lequel le code d'identification unique est configuré pour être lu par un lecteur codes-barres ; ou
dans lequel le code d'identification unique est intégré à une RFID.

12. Ensemble capteur implantable (10) selon la revendication 1, comprenant en outre un dispositif de mémoire pour stocker les données de capteur relatives à la ou aux caractéristiques.

13. Ensemble capteur implantable (10) selon la revendication 1, dans lequel l'ensemble de circuits de communications (35) est configuré pour communiquer sans fil avec le ou les dispositifs externes par l'intermédiaire d'un protocole Bluetooth^{™}, du WiFi, du protocole ZigBee, du service de communication pour implants médicaux (« MICS »), du service de radiocommunication pour dispositifs médicaux (« MedRadio ») ou de la téléphonie cellulaire.

14. Trousse, comprenant :
l'ensemble capteur implantable (10) selon la revendication 1 ; et
un système de pose (45) configuré pour poser l'ensemble capteur dans le passage corporel du patient, de préférence
dans laquelle le système de pose (45) est un cathéter à ballonnet ; ou
dans laquelle le système de pose (45) comprend une gaine conçue pour recouvrir les premier et second ancrages (15) lors de la pose de l'ensemble capteur implantable dans le passage corporel du patient.
